# EUROPÄISCHE PATENTSCHRIFT

(11) **EP 4 003 463 B1**
(45) Veröffentlichungstag und Bekanntmachung des Hinweises auf die Patenterteilung: **09.07.2025**
(21) Anmeldenummer: 20739951.0
(22) Anmeldetag: 09.07.2020
(51) Int. Cl.: A61M 5/20, A61M 5/30, A61M 5/31

(54) **VORRICHTUNG ZUM APPLIZIEREN EINES FLUIDS**
DEVICE FOR ADMINISTERING A FLUID
DISPOSITIF D'APPLICATION DE FLUIDE

(30) Priorität: 04.09.2019 DE 102019123733
(43) Veröffentlichungstag der Anmeldung: 01.06.2022
(73) Patentinhaber: Henke-Sass, Wolf GmbH, 78532 Tuttlingen (DE)
(72) Erfinder: ALTERMANN, Frank, 78532 Tuttlingen (DE); SEEH, Daniel, 78194 Immendingen (DE); SAUTER, Robin, 78532 Tuttlingen (DE); WIEDMANN, Maikel, 78194 Immendingen (DE); SCHMIDT, Anika, 78567 Fridingen (DE)
(74) Vertreter: Patentanwälte Geyer, Fehners & Partner mbB
(86) Internationale Anmeldenummer: PCT/EP2020/069453
(87) Internationale Veröffentlichungsnummer: WO 2021/043474

(56) Entgegenhaltungen:
- WO-A1-2007/140610
- WO-A1-2019/185604
- US-A- 3 057 349
- US-A- 3 973 697
- US-A- 5 480 381
- US-A1- 2011 224 613
- US-B2- 9 402 961

## Beschreibung

Die vorliegende Erfindung betrifft eine Vorrichtung zum Applizieren eines Fluids mit den Merkmalen des Oberbegriffs des Anspruches 1, die beispielsweise als nadellose Selbstfüllerspritze ausgebildet sein kann, mit der Tieren ein flüssiges Medikament, ein flüssiges Arzneimittel, ein flüssiger Impfstoff oder dergleichen intramuskulär verabreicht werden kann.

Eine solche Vorrichtung zum Applizieren eines Fluids (die z.B. aus der US 3 057 349 A bekannt ist) soll einerseits möglichst leicht und somit für einen Benutzer lange mit einer Hand tragbar sein und gleichzeitig das gewünschte nadellose intramuskuläre Injizieren ermöglichen.

Aufgabe der Erfindung ist es daher, eine solche Vorrichtung zum Applizieren eines Fluids bereitzustellen.

Die Erfindung ist im Anspruch 1 definiert. Vorteilhafte Ausgestaltungen sind in den abhängigen Ansprüchen angegeben.

Die erfindungsgemäße Vorrichtung zum Applizieren eines Fluids umfasst einen Zylinder, der ein offenes Abgabeende aufweist, einen im Zylinder zwischen einer vorderen und hinteren Endposition verschiebbaren Kolben, der mit einer Kolbenstange verbunden ist, die entlang einer ersten Richtung über ein dem offenen Abgabeende entgegengesetztes hinteres Ende des Zylinders heraussteht, ein das offene Abgabeende verschließendes Rückschlagventil (das als Auslassventil wirkt) und eine mit der Kolbenstange verbundene Spannvorrichtung. Die Spannvorrichtung kann die Kolbenstange in einem Spannvorgang entlang der ersten Richtung bewegen, bis der Kolben in seiner hinteren Endposition steht, um den Zylinder dadurch mit dem zu applizierenden Fluid zu befüllen und die Kolbenstange zum offenen Abgabeende hin vorzuspannen. Dazu kann die Vorrichtung einen Anschluss aufweisen, der im Zylinder mündet. An den Anschluss kann z.B. ein Schlauch oder ein Behälter mit dem zu applizierenden Fluid befestigbar sein und für den Einsatz der Vorrichtung befestigt werden. Bevorzugt kann der Anschluss ein Rückschlagventil aufweisen, das als Einlassventil ausgebildet ist und beim Spannvorgang öffnet und beim Applizieren des Fluids schließt. Entsprechend schließt das Auslassventil beim Spannvorgang und öffnet beim Applizieren des Fluids.

Die Spannvorrichtung kann ferner, wenn der Kolben in seiner hinteren Endposition steht, die Kolbenstange in einem Abgabevorgang freigeben, so dass der Kolben aufgrund der anliegenden Vorspannung entgegen der ersten Richtung zum offenen Abgabeende hin bewegt und dabei Fluid in dem Zylinder über das Rückschlagventil zum Applizieren abgegeben wird.

Die Spannvorrichtung weist eine mittels eines Motors drehbare Rampe mit einer sich entlang einer Schraubenlinie erstreckenden Rampenbahn auf, wobei die Rampenbahn von einem ersten Plateau entlang eines Steigungsbereiches zu einem zweiten Plateau ansteigt und vom zweiten Plateau über eine Sprungflanke zum ersten Plateau abfällt, wobei die Rampenbahn einen das zweite Plateau und die Sprungflanke verbindenden Übergangsbereich aufweist. Die Spannvorrichtung weist ferner eine die Rampenbahn kontaktierende Walze, die in einem Mitnehmer, der mit der Kolbenstange verbunden ist, drehbar gelagert ist, auf, so dass bei Drehung der Rampe entlang einer ersten Drehrichtung die Rampenbahn unter der sich dadurch drehenden Walze durchläuft. Für den Spannvorgang kann die Rampenbahn entlang der ersten Drehrichtung so gedreht werden, dass die Walze auf dem Steigungsbereich bis zum zweiten Plateau läuft und dadurch der Kolben in seine hintere Endposition bewegt wird. Für den Abgabevorgang kann die Spannvorrichtung die Rampenbahn ausgehend von einem Kontakt der Walze mit dem zweiten Plateau entlang der ersten Drehrichtung drehen, bis die Walze über den Übergangsbereich läuft und aufgrund der Vorspannung zum ersten Plateau hin beschleunigt, wodurch der Kolben zum offenen Abgabeende hin bewegt wird.

Die erfindungsgemäße Vorrichtung ist bevorzugt als Selbstfüllerspritze zum nadellosen Applizieren (insbesondere intramuskulär) bei Tieren und/oder Menschen ausgebildet.

Erfindungsgemäß kann der Motor über eine Kupplung mit der Rampe verbunden sein, wobei die Kupplung zum Drehen der Rampe das vom Motor bereitgestellte Drehmoment in der ersten Drehrichtung überträgt und dabei einen Freilauf entgegen der ersten Drehrichtung bereitstellt, der so ausgelegt ist, dass er mindestens einen Drehwinkelbereich abdeckt, der dem Übergangsbereich entspricht.

Die Kupplung kann so ausgebildet sein, dass der Freilauf einen Drehwinkelbereich abdeckt, der nicht mehr als das Doppelte des Übergangsbereichs entspricht. Der Freilauf kann insbesondere den Drehwinkelbereich abdecken, der um 10%, 20%, 30%, 40%, 50%, 60%, 70%, 80% oder 90% größer ist als der Übergangsbereich.

Die Kupplung kann ein erstes Kupplungsteil, das mit dem Motor verbunden ist, und ein zweites Kupplungsteil, das mit der Rampe verbunden ist, aufweisen. Eines der beiden Kupplungsteile kann ein vorstehendes Eingriffselement und das andere der beiden Kupplungsteile eine Ausnehmung, in die das Eingriffselement hineinsteht, aufweisen. Die Ausdehnung des Eingriffselementes in der ersten Drehrichtung kann um mindestens den den Übergangsbereich abdeckenden Drehwinkelbereich kleiner sein als die Ausdehnung der Ausnehmung in der ersten Drehrichtung. Somit ist die Ausdehnung der Ausnehmung in der ersten Drehrichtung größer als die Ausdehnung des Eingriffselementes in der ersten Drehrichtung, wodurch der gewünschte Freilauf bereitgestellt ist.

Zwischen einer Seitenfläche des Eingriffselementes und einer Seitenfläche der Ausnehmung, die in der ersten Drehrichtung zueinander weisen, kann eine Feder angeordnet sein. Insbesondere kann zwischen allen gegenüberliegenden Seitenflächen von Eingriffselement und Ausnehmung eine Feder angeordnet sein. Die Feder/Federn kann/können am Eingriffselement befestigt sein.

Die Feder/Federn kann/können als Druckfedern ausgebildet sein. Insbesondere können sie als Tellerfedern realisiert sein.

Das Eingriffselement kann als Steg ausgebildet sein.

Das erste Kupplungsteil kann das Eingriffselement aufweisen. Ferner kann die Rampe einen Boden als das zweite Kupplungsteil umfassen, wobei im Boden die Ausnehmung ausgebildet ist.

Das eine der beiden Kupplungsteile kann mehrere vorstehende Eingriffselemente, die in der ersten Richtung voneinander beabstandet sind, aufweisen. Das andere der beiden Kupplungsteile kann mehrere Ausnehmungen aufweisen, in die die Eingriffselemente einstehen. Die Ausdehnung jedes Eingriffselementes in der ersten Drehrichtung ist mindestens um den den Übergangsbereich abdeckenden Drehwinkelbereich kleiner als die Ausdehnung der entsprechenden Ausnehmung in der ersten Drehrichtung.

Erfindungsgemäß kann die Rampenbahn auf der Stirnseite einer sich entlang einer Kreisbahn erstreckenden Wandung verlaufen, wobei eine die Rampenbahn, den Mitnehmer und die Walze übergreifende Abdeckung vorgesehen ist, die mindestens einen sich entgegen der ersten Richtung erstreckenden Abstreifer aufweist, der sich innerhalb der Wandung bis zur Innenseite der Wandung erstreckt und so in der Innenseite befindliches Schmiermittel von der Innenseite abstreift.

Die Abdeckung kann mehrere sich entgegen der ersten Richtung erstreckende Abstreifer aufweisen, die sich jeweils innerhalb der Wandung in Richtung zur Innenseite der Wandung erstrecken und so in der Innenseite befindliches Schmiermittel von der Innenseite abstreifen, wobei die Abstreifer entlang der ersten Richtung voneinander beabstandet sind.

Die Abstreifer können sich in ihrer Länge entgegen der ersten Richtung unterscheiden.

Ferner können sich die Abstreifer in ihrer Ausdehnung in Richtung zur Innenseite hin unterscheiden.

Der bzw. die Abstreifer kann bzw. können an einem kegelstumpfförmigen Mittelteil ausgebildet sein. Insbesondere können sie sich radial vom kegelstumpfförmigen Mittelteil erstrecken. Das kegelstumpfförmige Mittelteil kann sich entgegen der ersten Richtung erstrecken. Insbesondere kann sich das kegelstumpfförmige Mittelteil bis zum Boden der Rampe erstrecken.

Das Mittelteil kann auch jede andere Form aufweisen. Insbesondere kann es zylinderförmig ausgebildet sein.

Erfindungsgemäß kann die Kolbenstange über ein Gelenk mit dem Mitnehmer verbunden sein.

Insbesondere kann zur Ausbildung des Gelenks das dem Kolben abgewandte Ende der Kolbenstange abgerundet und bewegbar in einem Bett gelagert sein.

Das Bett kann an einem Verbindungsteil ausgebildet sein, das mittels einer in das abgerundete Ende eingeschraubten Schraube gegen das abgerundete Ende der Kolbenstange drückt. Das Bett kann durch eine gekrümmte Seite einer Beilagscheibe (oder einer Ausgleichsscheibe) ausgebildet sein.

Ferner kann das Gelenk zwei aufeinander angeordnete Beilagscheiben (oder Ausgleichsscheiben) aufweisen, deren einander zugewandte Seiten gekrümmt ausgebildet sind, so dass sie bei Drehung der Kolbenstange gegeneinander bewegt werden. Die beiden Beilagscheiben können auf einer vom abgerundeten Ende der Kolbenstange abgewandten Seite des Verbindungsteils angeordnet sein.

Das Gelenk kann als Drehgelenk und/oder als Gelenk mit genau einem Freiheitsgrad ausgebildet sein.

Das Gelenk kann eine translatorische Bewegung (bevorzugt genau eine translatorische Bewegung) quer zur Längsrichtung der Kolbenstange zulassen.

Erfindungsgemäß kann die Vorrichtung zum Applizieren genau einen Zylinder mit genau einem Kolben und genau einer Kolbenstange aufweisen, wobei die Spannvorrichtung zwei zueinander parallel verlaufende Schraubenfedern aufweist, die beide, wenn der Kolben in seiner hinteren Endposition steht, zur anliegenden Vorspannung beitragen.

Die zwei Schraubenfedern können quer zu ihrer Längsrichtung voneinander beabstandet angeordnet sein und/oder gleiche Abmessungen aufweisen.

Insbesondere können die Schraubenfedern so angeordnet sein, dass ihre Längsrichtungen parallel zur Längsrichtung der Kolbenstange sind.

Die Schraubenfedern können als Druckfedern ausgebildet sein.

Die Kolbenstange kann über ein Verbindungsteil mit zwei Führungsstangen verbunden sein, wobei sich jede Führungsstange innerhalb einer der Schraubenfedern erstreckt.

Die Spannvorrichtung kann mindestens drei zueinander parallel verlaufende Schraubenfedern aufweisen. Insbesondere können die Schraubenfedern in einer Ebene senkrecht zur Längsrichtung der Schraubenfedern symmetrisch zum Motor angeordnet sein.

Erfindungsgemäß kann die Vorrichtung zum Applizieren eines Fluids ein den Zylinder und das offene Abgabeende aufweisendes Vorderteil und ein die Spannvorrichtung aufweisendes Hinterteil umfassen, wobei das Vorderteil und das Hinterteil aus unterschiedlichen Materialien gebildet sind.

Das Material des Vorderteils kann Titan, Stahl oder Kunststoff umfassen und das Material des Hinterteils kann Titan, Aluminium, Magnesium oder Kunststoff umfassen.

Die Vorrichtung kann ein das Vorderteil und das Hinterteil umschließendes Gehäuse aufweisen, wobei ein Abschnitt des Vorderteils aus dem Gehäuse heraussteht.

Erfindungsgemäß kann die Vorrichtung eine Dosiereinstelleinrichtung mit einem Abstandshalter und einer Bewegungseinheit umfassen, wobei die Bewegungseinheit den Abstandshalter, wenn der Kolben in seiner hinteren Endposition steht, von einer neutralen Position, in der der Abstandshalter nicht zwischen dem Mitnehmer und dem Zylinder positioniert ist, in eine aktive Position zwischen dem Mitnehmer und dem Zylinder bewegen kann, so dass der Mitnehmer, nachdem die Walze den Übergangsbereich überlaufen hat, durch den Abstandshalter gestoppt wird und somit der Kolbenhub bei der Bewegung des Kolbens zum offenen Abgabeende kürzer ist im Vergleich zu dem Fall, bei der Abstandshalter in seiner neutralen Position ist.

Der Abstandshalter kann eine Gewindebohrung aufweisen, in die eine Gewindestange einsteht, die zum Bewegen des Abstandshalters zwischen seiner neutralen Position und seiner aktiven Position gedreht wird.

Der Abstandshalter kann so geführt sein, dass der Abstandshalter nur in einer Ebene senkrecht zur Kolbenstange bewegbar ist.

Der Abstandshalter kann so ausgebildet sein, dass, wenn der Mitnehmer durch den Abstandshalter gestoppt ist, die Walze nicht in Kontakt mit dem Abstandshalter steht.

Der Abstandshalter kann einen ersten Anschlagbereich und einen zweiten Anschlagbereich für den Mitnehmer aufweisen, wobei die Ausdehnung des Abstandshalters entlang der ersten Richtung für den ersten Anschlagbereich kleiner ist als für den zweiten Anschlagbereich, so dass unterschiedliche Kolbenhubverkürzungen einstellbar sind, je nachdem ob der erste oder zweite Anschlagbereich in die aktive Position des Abstandshalters bewegt ist.

Natürlich kann der Abstandshalter auch drei oder mehr Anschlagbereiche aufweisen, wobei die Ausdehnung des Abstandshalters entlang der ersten Richtung für die Anschlagbereiche unterschiedlich ist, so dass unterschiedliche Kolbenhubverkürzungen einstellbar sind, je nachdem welcher Anschlagbereich in die aktive Position des Abstandshalters bewegt ist.

Erfindungsgemäß kann die Vorrichtung eine Steuereinheit aufweisen, die eine Messung einer Kenngröße während eines Spann- und/oder Abgabevorgangs durchführt und daraus durch Vergleichen mit mindestens einem Vorgabewert ermittelt, ob der Spann- und/oder Abgabevorgang bestimmungsgemäß erfolgt ist. Insbesondere kann die Messung der Kenngröße während des Abgabevorgangs und des vorhergehenden Spannvorgangs durchgeführt werden und daraus durch Vergleichen mit dem mindestens einen Vorgabewert ermittelt, ob sowohl der Spann- als auch der Abgabevorgang bestimmungsgemäß erfolgt sind.

Als Kenngröße kann die Stromaufnahme des Motors, die auf die Vorrichtung zum Applizieren wirkende Beschleunigung und/oder der Schall (bzw. die Geräusche; z.B. Frequenzspektrum, Frequenz(en), Tonhöhe, Energie und/oder Lautstärke) gemessen werden.

Es kann ein zeitlicher Sollverlauf der Stromaufnahme mit einer unteren Grenze und einer oberen Grenze als der mindestens eine Vorgabewert vorgegeben sein, wobei die Steuereinheit den Spannvorgang als bestimmungsgemäß bestimmt, wenn die gemessene Stromaufnahme während des gesamten Spannvorgangs nicht kleiner als die untere Grenze und nicht größer als die obere Grenze ist.

Es kann ein zeitlicher Sollverlauf der Beschleunigung mit einer oberen Grenze als der mindestens eine Vorgabewert vorgegeben sein, wobei die Steuereinheit den Abgabevorgang als bestimmungsgemäß bestimmt, wenn die gemessene Beschleunigung während des gesamten Abgabevorgang nicht größer als die obere Grenze ist.

Es kann eine erste obere sowie eine erste untere Sollfrequenz und/oder eine erste obere sowie eine erste untere Sollamplitude als der mindestens eine Vorgabewert vorgegeben sein, wobei die Steuereinheit den Abgabevorgang als bestimmungsgemäß bestimmt, wenn eine Hauptfrequenz des gemessenen Frequenzspektrums zwischen der ersten oberen und der ersten unteren Sollfrequenz liegt und/oder die Amplitude der Hauptfrequenz des gemessenen Frequenzspektrums zwischen der ersten oberen und der ersten unteren Sollamplitude liegt.

Unter der Hauptfrequenz wird hier insbesondere die Frequenz des gemessenen Frequenzspektrums verstanden, die die größte Amplitude aufweist. Die Hauptfrequenz ist üblicher Weise die Frequenz, die die Tonhöhe bestimmt.

Die erste obere Sollfrequenz kann um 0,5%, 1%, 1,5%, 2%, 2,5%, 3%, 3,5%, 4%, 4,5%, 5%, 6%, 7%, 8%, 9%, 10%, 12%, 14% oder 15% größer sein als eine vorgegebene erste Sollhauptfrequenz. Ferner kann die erste untere Sollfrequenz um 0,5%, 1%, 1,5%, 2%, 2,5%, 3%, 3,5%, 4%, 4,5%, 5%, 6%, 7%, 8%, 9%, 10%, 12%, 14% oder 15% kleiner sein als die vorgegebene erste Sollhauptfrequenz.

Die erste obere Sollamplitude kann um 0,5%, 1%, 1,5%, 2%, 2,5%, 3%, 3,5%, 4%, 4,5%, 5%, 6%, 7%, 8%, 9%, 10%, 12%, 14% oder 15% größer sein als eine vorgegebene erste Sollhauptamplitude. Ferner kann die erste untere Sollamplitude um 0,5%, 1%, 1,5%, 2%, 2,5%, 3%, 3,5%, 4%, 4,5%, 5%, 6%, 7%, 8%, 9%, 10%, 12%, 14% oder 15% kleiner sein als die vorgegebene erste Sollhauptamplitude.

Ferner kann eine zweite obere sowie eine zweite untere Sollfrequenz und/oder eine zweite obere sowie eine zweite untere Sollamplitude als der mindestens eine Vorgabewert vorgegeben sein, wobei die Steuereinheit den Abgabevorgang als bestimmungsgemäß bestimmt, wenn eine erste Nebenfrequenz des gemessenen Frequenzspektrums zwischen der zweiten oberen und der zweiten unteren Sollfrequenz liegt und/oder die Amplitude der ersten Nebenfrequenz des gemessenen Frequenzspektrums zwischen der zweiten oberen und der zweiten unteren Sollamplitude liegt.

Unter der ersten Nebenfrequenz wird hier insbesondere die Frequenz des gemessenen Frequenzspektrums verstanden, die die zweithöchste Amplitude und somit nach der Hauptfrequenz die größte Amplitude aufweist.

Die zweite obere Sollfrequenz kann um 0,5%, 1%, 1,5%, 2%, 2,5%, 3%, 3,5%, 4%, 4,5%, 5%, 6%, 7%, 8%, 9%, 10%, 12%, 14% oder 15% größer sein als eine vorgegebene erste Sollnebenfrequenz. Ferner kann die zweite untere Sollfrequenz um 0,5%, 1%, 1,5%, 2%, 2,5%, 3%, 3,5%, 4%, 4,5%, 5%, 6%, 7%, 8%, 9%, 10%, 12%, 14% oder 15% kleiner sein als die vorgegebene erste Sollnebenfrequenz.

Die zweite obere Sollamplitude kann um 0,5%, 1%, 1,5%, 2%, 2,5%, 3%, 3,5%, 4%, 4,5%, 5%, 6%, 7%, 8%, 9%, 10%, 12%, 14% oder 15% größer sein als eine vorgegebene erste Sollnebenamplitude. Ferner kann die zweite untere Sollamplitude um 0,5%, 1%, 1,5%, 2%, 2,5%, 3%, 3,5%, 4%, 4,5%, 5%, 6%, 7%, 8%, 9%, 10%, 12%, 14% oder 15% kleiner sein als die vorgegebene erste Sollnebenamplitude.

Natürlich kann eine zweite, dritte, vierte, fünfte und/oder weitere Nebenfrequenzen (deren Amplituden jeweils kleiner sind) in gleicher Weise gemessen und berücksichtigt werden, um den Abgabevorgang zu bewerten.

den Abgabevorgang als bestimmungsgemäß bestimmt, wenn die Hauptfrequenz des gemessenen Frequenzspektrums kleiner ist als die Sollfrequenz und/oder die Amplitude der Hauptfrequenz des gemessenen Frequenzspektrums größer ist als die Sollamplitude. Unter der Hauptfrequenz wird hier insbesondere die Frequenz des gemessenen Frequenzspektrums verstanden, die die größte Amplitude aufweist. Die Hauptfrequenz ist üblicher Weise die Frequenz, die die Tonhöhe bestimmt.

Als Kenngröße kann die Zeitdauer des Spannvorgangs gemessen werden.

Als Vorgabewert kann eine erste Sollzeitdauer vorgegeben sein, wobei die Steuereinheit den Spannvorgang als bestimmungsgemäß bestimmt, wenn die gemessene Zeitdauer größer ist als die erste Sollzeitdauer.

Als Vorgabewert kann eine zweite Sollzeitdauer vorgegeben sein, wobei die Steuereinheit den Spannvorgang als bestimmungsgemäß bestimmt, wenn die gemessene Zeitdauer kleiner ist als die zweite Sollzeitdauer.

Ferner kann als Kenngröße der überstrichene Drehwinkel der Rampenbahn entlang der ersten Drehrichtung während des Spannvorgangs gemessen werden.

Als Vorgabewert kann ein Solldrehwinkel vorgegeben sein, wobei die Steuereinheit den Spannvorgang als bestimmungsgemäß bestimmt, wenn der gemessene überstrichene Drehwinkel größer ist als der Solldrehwinkel.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine perspektivische Ansicht eines Ausführungsbeispiels der erfindungsgemäßen Applizierungsvorrichtung 1;
- Fig. 2: eine Vorderansicht der Zylinder-Kolben-Anordnung 10 der Applizierungsvorrichtung 1;
- Fig. 3: eine Schnittansicht der Zylinder-Kolben-Anordnung 10 gemäß der Schnittlinie A-A in Fig. 2;
- Fig. 4: eine Schnittansicht der Zylinder-Kolben-Anordnung 10 entlang der Schnittlinie B-B in Fig. 3;
- Fig. 5: eine Schnittansicht der Zylinder-Kolben-Anordnung 10 gemäß der Schnittlinie C-C in Fig. 4;
- Fig. 6: eine isometrische Ansicht der Zylinder-Kolben-Anordnung 10, wobei die Vorrichtung gespannt ist und der Kolben in seiner hinteren Endposition steht;
- Fig. 7: eine isometrische Ansicht der Zylinder-Kolben-Anordnung 10, wobei der Kolben in seiner vorderen Endposition steht;
- Fig. 8: ein Diagramm zur Darstellung des Verlaufs der Rampenbahn 41, wobei entlang der x-Achse der Drehwinkel α und entlang der y-Achse der Hub entlang der Längsachse der Kolbenstange 25 aufgetragen ist;

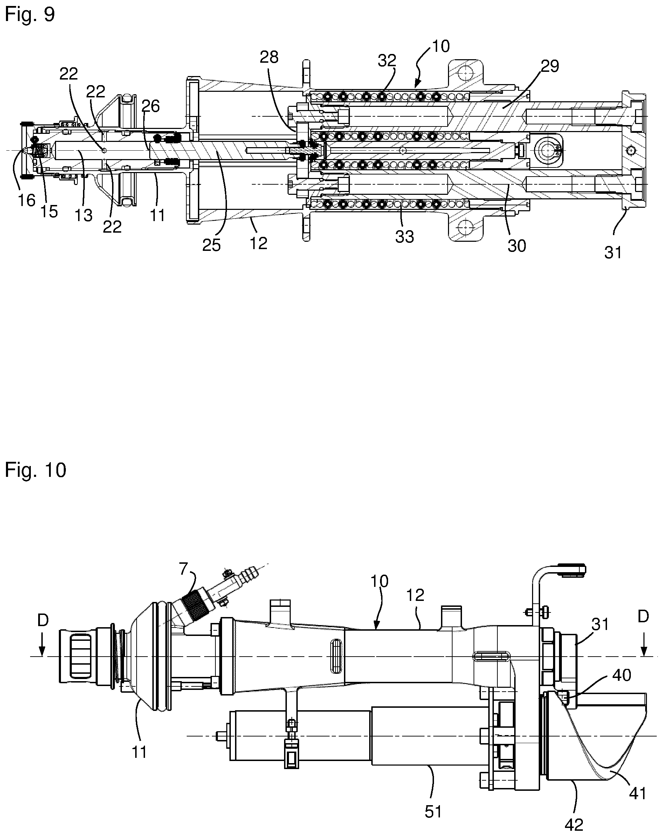

- Fig. 9: eine Schnittansicht der Kolben-Zylinder-Anordnung 10 im gespannten Zustand gemäß Fig. 6;
- Fig. 10: eine Vorderansicht der Kolben-Zylinder-Anordnung 10, bei der der Kolben in seiner vorderen Endposition steht;
- Fig. 11: eine Schnittansicht der Kolben-Zylinder-Anordnung 10 gemäß der Schnittlinie D-D in Fig. 10;
- Fig. 12A-12C: Darstellungen zur Erläuterung der Kräfte, wenn die Walze 40 den Übergangsbereich 46 zur Sprungflanke 47 hin überläuft;
- Fig. 13: eine Vorderansicht des Bodens 60 der Rampe 42;
- Fig. 14: eine isometrische Ansicht des mit dem Motor 51 drehfest verbundenen Kupplungsteils 66;

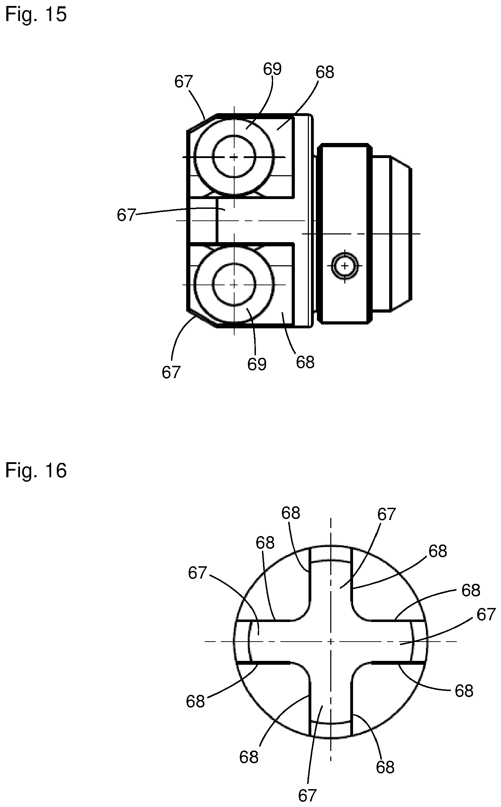

- Fig. 15: eine Seitenansicht des Kupplungsteils 66;
- Fig. 16: eine Vorderansicht des Kupplungsteils 66;
- Fig. 17: eine Vorderansicht des in die Ausnehmung 61 im Boden 60 eingesetzten Kupplungsteils 66;
- Fig. 18 und 19: Darstellungen gemäß Fig. 17 zur Erläuterung des durch die Kupplung 50 bereitgestellten Freilaufs;
- Fig. 20: eine schematische Vorderansicht der räumlichen Anordnung der Federn 32 und 33 und des Motors 51;
- Fig. 21: eine Vergleichsansicht der Anordnung einer Feder und des Motors einer herkömmlichen Applizierungsvorrichtung;
- Fig. 22 und 23: weitere Ansichten weiterer Ausführungsbeispiele für die räumliche Anordnung von Feder und Motor bei der erfindungsgemäßen Applizierungsvorrichtung 1;
- Fig. 24: eine Ansicht des Vorderteils 11 und Hinterteils 12 im verbundenen Zustand;
- Fig. 25: eine Ansicht des Vorderteils 11 und des Hinterteils 12 im nicht verbundenen Zustand;
- Fig. 26: ein vergrößerter Detailschnitt der Dosiereinstelleinrichtung 36;
- Fig. 27A: eine perspektivische Ansicht des Abstandshalters 70;
- Fig. 27B: eine schematische Darstellung eines weiteren Ausführungsbeispiels eines Abstandshalters 70;
- Fig. 28: eine perspektivische Darstellung der Abdeckung 35;
- Fig. 29: eine Vorderansicht der Abdeckung 35;

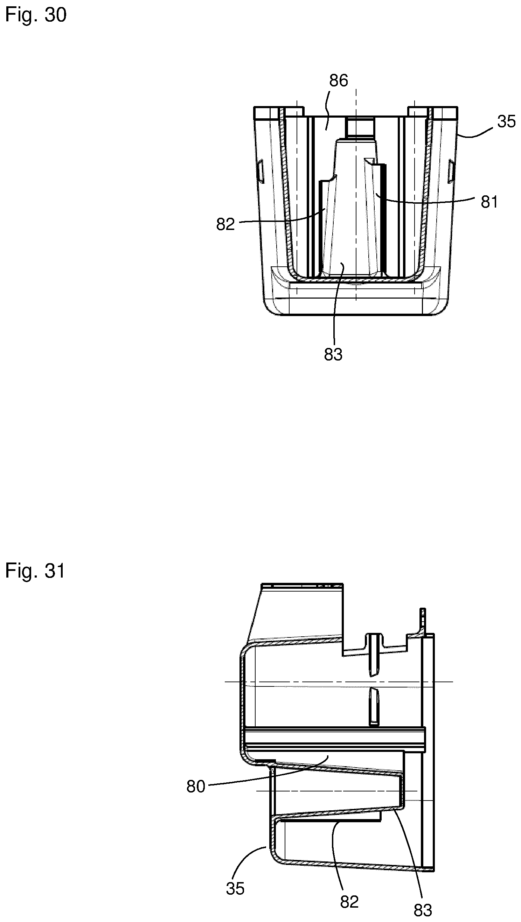

- Fig. 30: eine Schnittansicht entlang der Schnittlinie B-B in Fig. 29;
- Fig. 31: eine Schnittansicht entlang der Schnittlinie A-A in Fig. 29;

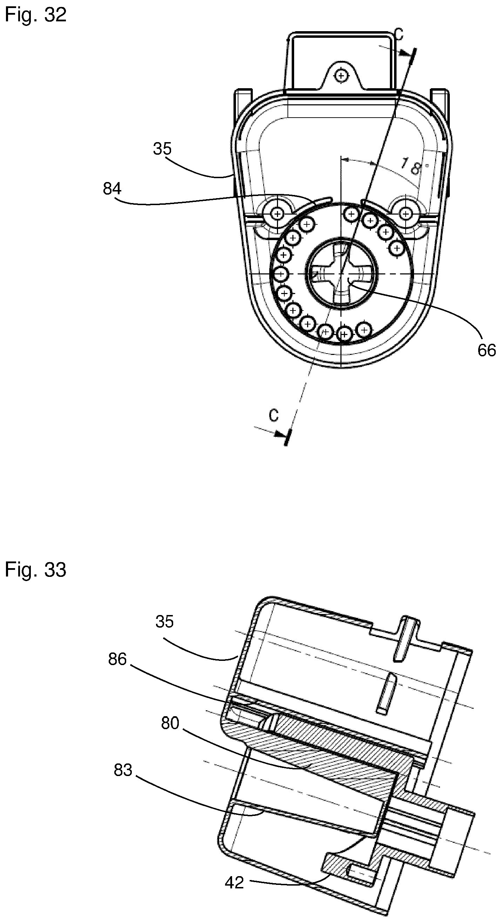

- Fig. 32: eine Vorderansicht der Abdeckung 31;
- Fig. 33: eine Schnittansicht der Abdeckung 35 entlang der Schnittlinie C-C in Fig. 32;
- Fig. 34: eine vergrößerte Schnittdarstellung des Vorderteils 11 samt Kolben 26 und Teil der Kolbenstange 25;
- Fig. 35: eine schematische Schnittdarstellung eines Einsatzes 96 für die Düse 16;
- Fig. 36: eine schematische Schnittdarstellung von Kolbenstange 25, Platte 28 sowie Führungsstangen 29 und 30 und Federn 32 und 33;
- Fig. 37: eine vergrößerte Darstellung des Details A von Fig. 36;
- Fig. 38 und 39: Diagramme mit Messwerten von gemessenen Beschleunigungswerten während des Spann- und Abgabevorgangs;
- Fig. 40 und 41: Diagramme mit Messwerten der gemessenen Stromaufnahme des Motors 51 während eines Spann- und Abgabevorgangs.

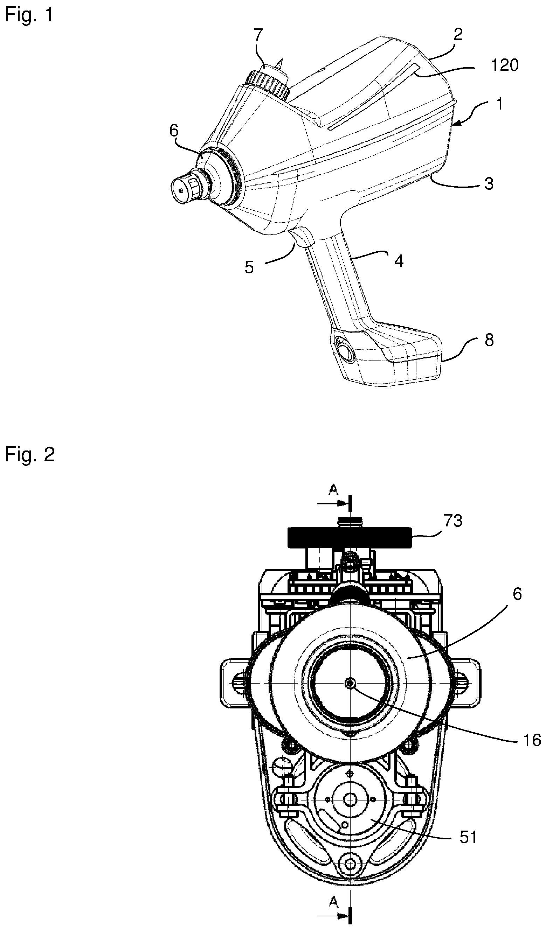

Bei dem in Fig. 1 gezeigten Ausführungsbeispiel umfasst die erfindungsgemäße Vorrichtung 1 zum Applizieren eines Fluids (z.B. einer Flüssigkeit) ein Gehäuse 2 mit einem Hauptabschnitt 3 und einem Griffabschnitt 4. Der Griffabschnitt 4 ist so ausgebildet, dass ein Benutzer die Vorrichtung 1 durch Umgreifen des Griffabschnittes halten kann. Des Weiteren weist der Griffabschnitt 4 einen Auslöser 5 zum Betätigen der Vorrichtung auf. Am vorderen Ende des Hauptabschnittes 3 ist ein Abgabebereich 6 ausgebildet. Des Weiteren weist die Vorrichtung 1 am oberen Bereich des Hauptabschnittes 3 einen Anschluss 7 auf, mit dem z.B. ein Schlauch oder ein Behälter verbunden werden kann. Über den Schlauch kann das zu applizierende Fluid zugeführt werden. In gleicher Weise kann im Behälter das zu applizierende Fluid enthalten sein.

Der Griffabschnitt 4 geht an seinem vom Hauptabschnitt 3 wegweisenden Ende in einen Fuß 8 über, in dem z.B. eine Stromversorgung (beispielsweise ein Akku) für die Vorrichtung 1 enthalten sein kann.

Die erfindungsgemäße Vorrichtung 1, die auch als Applizierungsvorrichtung 1 bezeichnet werden kann, ist bei dem hier beschriebenen Ausführungsbeispiel zum nadellosen Applizieren des Fluids bei einem Tier ausgebildet. Bei dem Applizieren handelt es sich bevorzugt um ein intramuskuläres Injizieren des Fluids, das z.B. ein Arzneimittel, ein Impfstoff oder dergleichen sein kann.

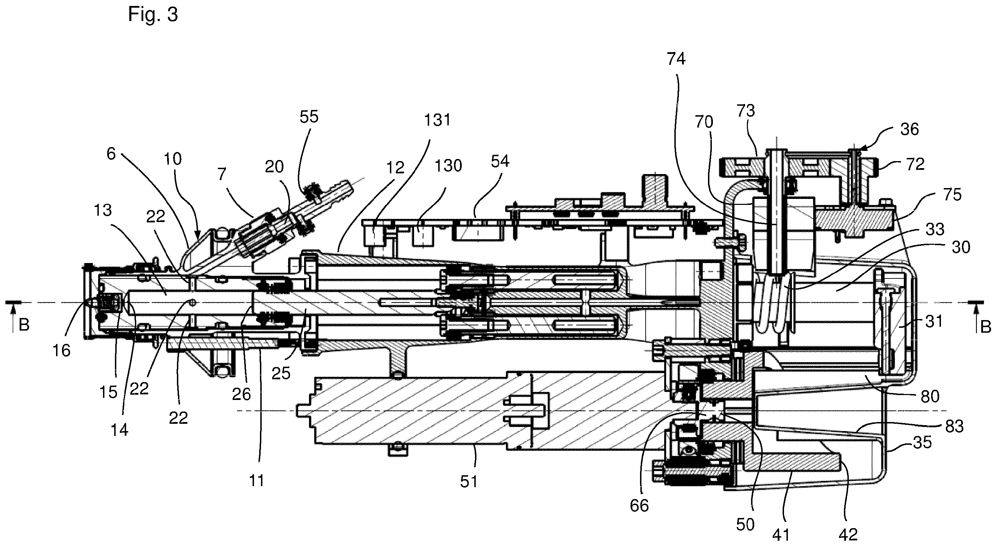

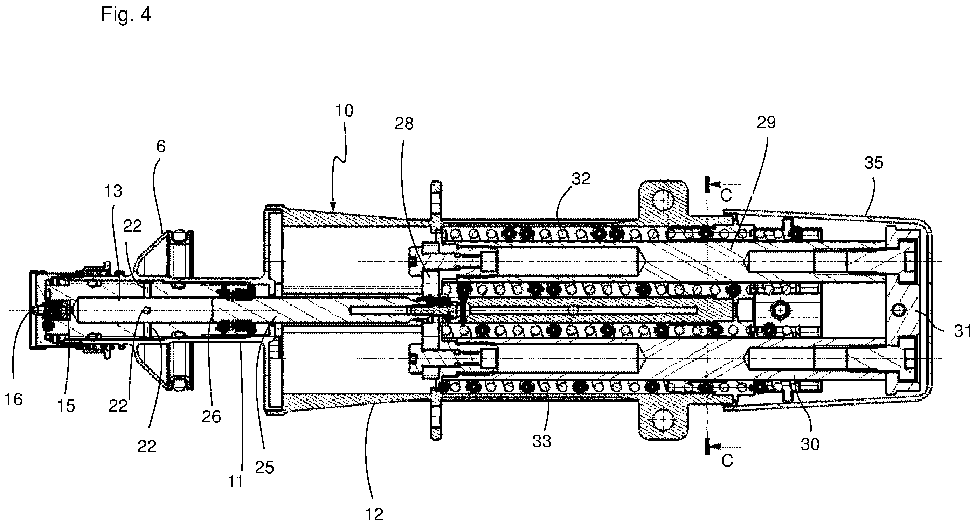

Die Applizierungsvorrichtung 1 weist eine nachfolgend noch im Detail beschriebene Zylinder-Kolben-Anordnung 10 (Fig. 3 und 4) auf und ist als Selbstfüllertyp derart ausgebildet, dass durch eine Kolbenbewegung zum Abgabebereich 6 hin ein Ausspritzen des Fluids bewirkt wird und durch eine entgegengesetzte Bewegung des Kolbens ein Auffüllen des Zylinders mit dem Fluid für den nächsten Ausspritzvorgang bewirkt wird.

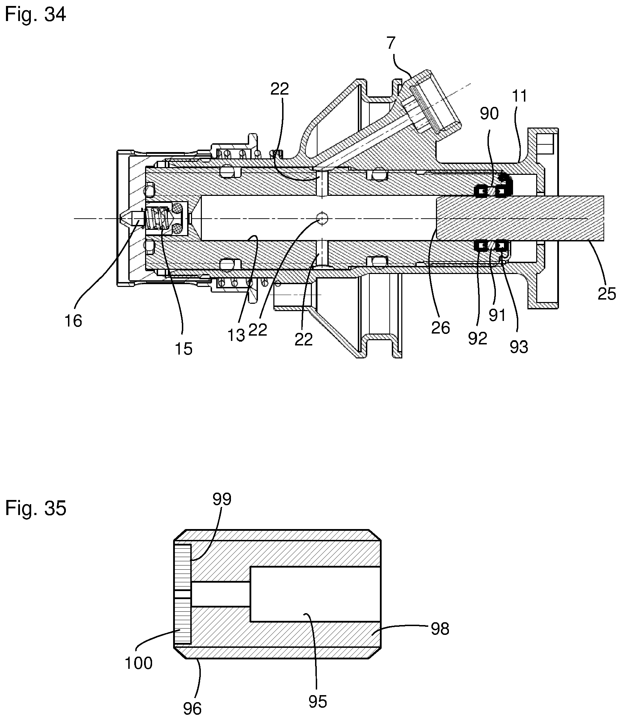

In Fig. 2 bis 5 ist die gesamte Zylinder-Kolben-Anordnung 10 ohne Gehäuse 2 dargestellt. Die Zylinder-Kolben-Anordnung 10 umfasst ein Vorderteil 11 und ein damit verbundenes Hinterteil 12. Das Vorderteil 11 umfasst einen Zylinder 13 zur Aufnahme des Fluids, der ein offenes Abgabeende 14 aufweist, in dem ein Rückschlagventil 15 sitzt, das in Fluidverbindung mit einer Düse 16 ist. Das Rückschlagventil 15 ist auch gut in der Darstellung gemäß Fig. 34 zu erkennen und ist so ausgebildet, dass eine Abgabe des Fluids aus dem Zylinder 13 über das Rückschlagventil 15 und die Düse 16 ermöglicht wird. Ein Ansaugen von Luft oder Flüssigkeit über die Düse und über das Rückschlagventil 15 ist nicht möglich. In dieser Richtung schließt das Rückschlagventil 15.

An dem Vorderteil 11 ist ferner der Anschluss 7 ausgebildet, in dem ein weiteres Rückschlagventil 20 (Fig. 3) sitzt, das eine Fluidverbindung vom Anschluss 7 zum Zylinder 13 ermöglicht und eine Fluidverbindung in entgegengesetzter Richtung sperrt. Der Anschluss 7 weist einen Kanal 21 auf, der über mehrere radiale Bohrungen 22 im Zylinder 13 mündet.

Das weitere Rückschlagventil 20 kann somit als Einlassventil und das Rückschlagventil 15 kann als Auslassventil bezeichnet werden.

Im Zylinder 13 ist eine Kolbenstange 25 mit einem an ihrem zum offenen Abgabeende 14 hin weisenden Ende ausgebildeten Kolben 26 geführt, wobei der Kolben 26 in den Schnittdarstellungen von Fig. 3 und 4 in seiner hinteren Endposition steht. In dieser Position ist der Zylinder 13 mit dem abzugebenden Fluid gefüllt.

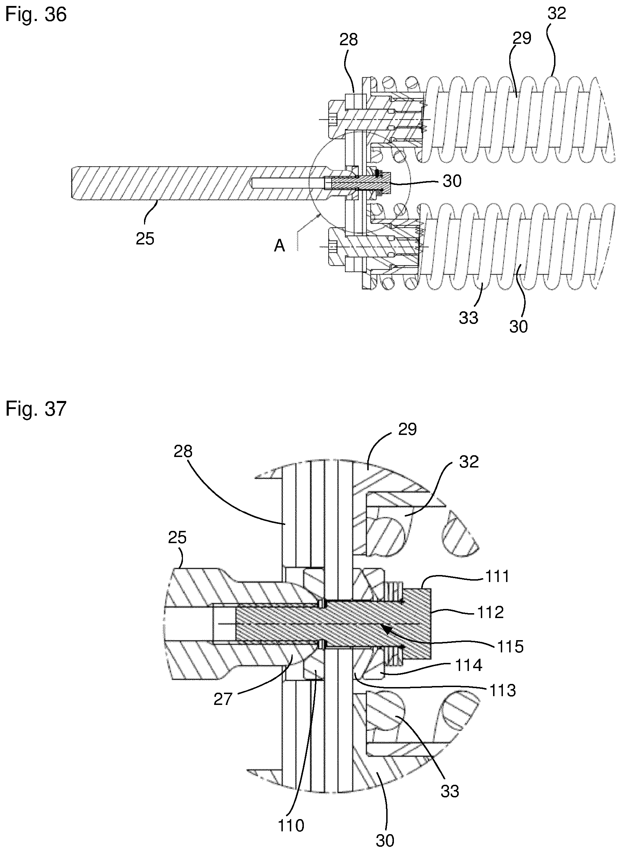

Das von dem offenen Abgabeende 14 wegweisende hintere Ende 27 (gut in Fig. 37 zu erkennen) der Kolbenstange 25 ist über eine Platte 28 mit einer ersten Führungsstange 29 und einer zweiten Führungsstange 30 verbunden, die sich parallel zueinander und parallel zur Kolbenstange 25 erstrecken und die im Hinterteil 12 geführt sind (Fig. 4). Die von der Platte 28 wegweisenden Enden der Führungsstangen 29 und 30 sind mit einem Mitnehmer 31 verbunden.

Ferner ist für jede Führungsstange 29 und 30 eine Druckfeder 32, 33 (z.B. Schraubenfeder) angeordnet, deren vordere Enden jeweils an der Platte 28 und deren hintere Enden jeweils an einem Anschlag 34 des Hinterteils 12 anliegen. In der in Fig. 3 und 4 gezeigten Stellung des Kolbens 26 sind die Federn 32, 33 gespannt.

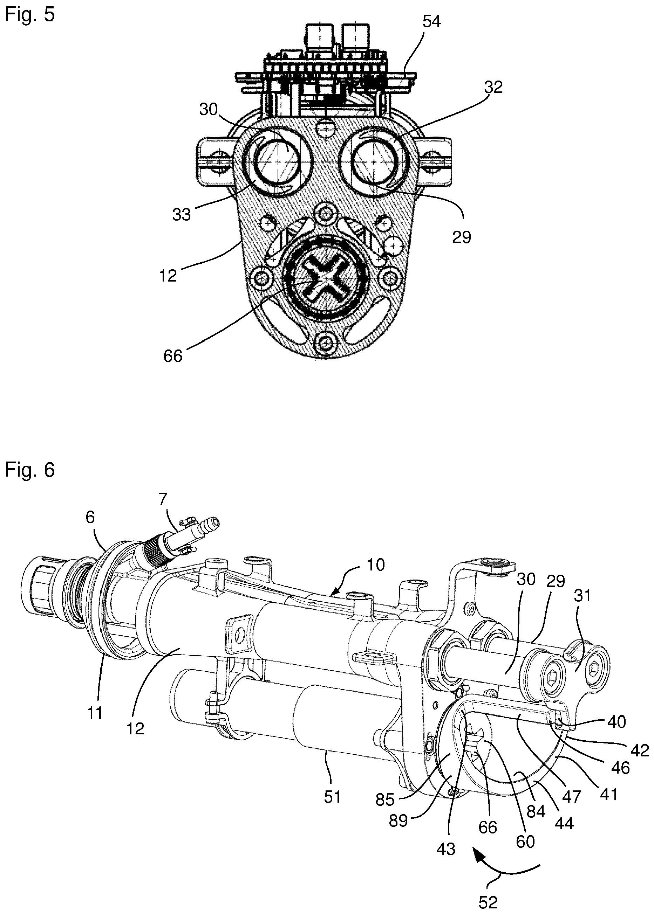

Am hinteren Ende des Hinterteils 12 ist eine Abdeckung 35 sowie eine Dosiereinstelleinrichtung 36 vorgesehen, die in der isometrischen Ansicht der Zylinder-Kolben-Anordnung 10 gemäß Fig. 6 nicht dargestellt sind, so dass der Mitnehmer 31 gut erkennbar ist. Der Mitnehmer 31 weist eine drehbar gelagerte Walze 40 auf, wobei sich die Drehachse der Walze 40 im Wesentlichen senkrecht zur Längsachse der Kolbenstange 25 erstreckt.

Die Walze 40 läuft auf einer Rampenbahn 41 einer sich unter der Walze 40 hindurch drehenden Rampe 42, wobei die Rampenbahn 41 eine einzige Windung aufweist, wie insbesondere Fig. 6 bis 8 zu entnehmen ist.

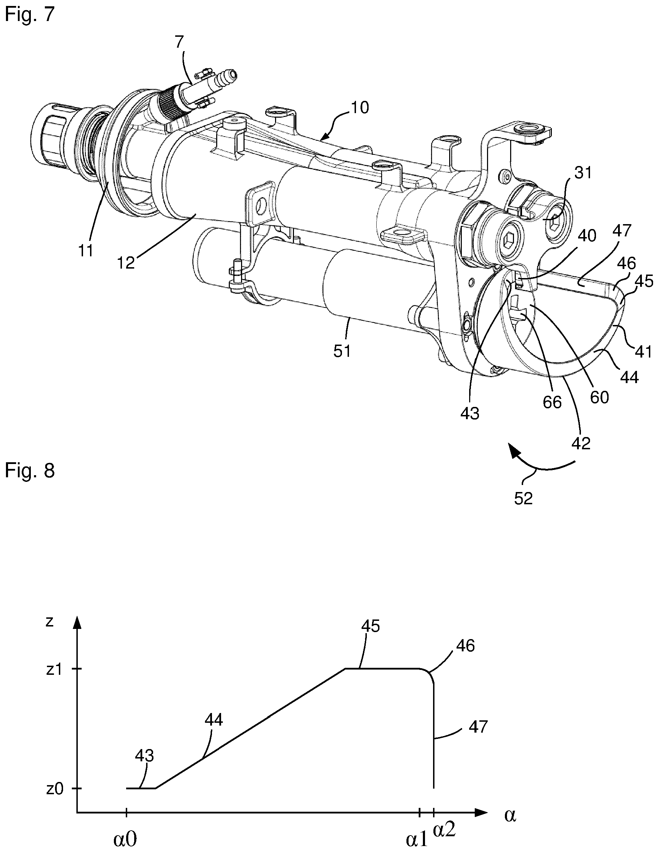

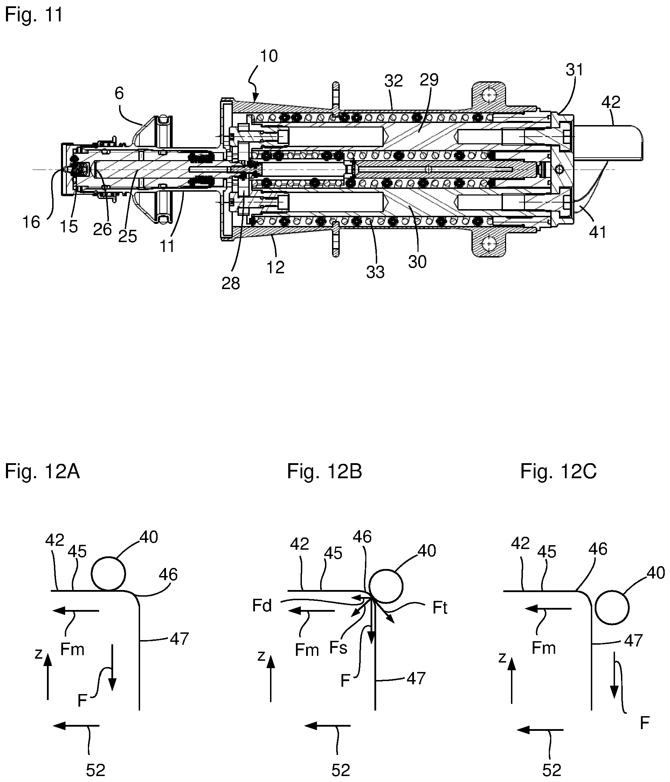

In Fig. 8 ist der Drehwinkel α gegenüber dem Gangunterschied z parallel zur Längsrichtung der Kolbenstange 25 aufgetragen, wobei davon ausgegangen wird, dass bei einem Drehwinkel von α0 = 0° die geringste Ganghöhe z0 vorliegt und der Kolben 26 somit in einer vorderen Endposition steht, in der sein Abstand zum offenen Abgabeende 14 minimal ist. Diese Stellung des Kolbens 26 ist beispielsweise in der Schnittdarstellung gemäß Fig. 11 gezeigt.

Die Rampenbahn 41 weist ein unteres Plateau 43 auf, an dem sich ein Steigungsbereich 44 anschließt, der bis zum oberen Plateau 45 verläuft. An das obere Plateau 45 schließt sich ein Übergangsbereich 46 an, der in eine Sprungflanke 47 (Drehwinkel α1) mündet, die wieder zum ersten Plateau 43 führt. Somit entspricht der Drehwinkelbereich von α0 bis α2 gleich 360°.

Die Sprungflanke 47 zeichnet sich dadurch aus, dass sie quasi senkrecht verläuft, da sie sich beim einem Drehwinkel (hier α2) von der Höhe z1 zur Höhe z0 erstreckt. Der Übergangsbereich 46 ist somit der Drehwinkelbereich, bei dem die Höhe z1 ausgehend vom oberen Plateau 45 kontinuierlich abnimmt, bis der Drehwinkel α2 (= Sprungflanke 47) erreicht ist. Somit deckt der Drehwinkelbereich von α1 zu α2 den Übergangsbereich 46 ab.

Die Rampe 42 ist über eine Kupplung 50 mit einem Motor 51 verbunden (Fig. 3), der die Rampe 42 in einer ersten Drehrichtung 52 dreht (Figuren 6 und 7). Wenn nun der Motor 51 die Rampe 42 ausgehend von der in Fig. 6 gezeigten Position, in der die Zylinder-Kolben-Anordnung 10 gespannt ist, weiter in der ersten Drehrichtung 52 dreht (da ein Benutzer den Auslöser 5 betätigt hat), läuft die Walze 40 über den Übergangsbereich 46 und fällt dann entlang der Sprungflanke 47 in Richtung zum unteren Plateau 43, da die gespannten Druckfedern 32 und 33 die Platte 28 in Richtung hin zum offenen Abgabeende 14 beschleunigen, wodurch die mit der Platte 28 verbundene Kolbenstange 25 ebenfalls zum vorderen Abgabeende 14 hin bewegt wird und dabei das im Zylinder 13 enthaltene Fluid über das Rückschlagventil 15 und die Düse 16 zur intramuskulären Injektion bei einem Tier ausgespritzt wird. Die Applizierungsvorrichtung 1 ist dabei so ausgelegt, dass das Fluid sicher die Haut durchdringt und in den darunter liegenden Muskel appliziert wird. Der Kolben 26 steht dann in seiner vorderen Endposition, wie z.B. in der Schnittdarstellung in Fig. 11 gezeigt ist. Die Applizierungsvorrichtung 1 ist bevorzugt so ausgelegt, dass in der vorderen Endposition des Kolbens 26 der Mitnehmer 31 am hinteren Ende des Hinterteils 12 anliegt, wodurch das hintere Ende des Hinterteils 12 einen Anschlag für den Mitnehmer 31 bildet. In dieser Position liegt noch ein gewünschter Mindestabstand zwischen der Walze 40 und der Rampenbahn 41 vor, so dass von der Walze 40 das untere Plateau 43 der Rampenbahn 41 nicht erreicht wird. Damit kann verhindert werden, dass die Walze 40 am Ende des Ausspritzvorgangs auf die Rampenbahn 41 trifft, was zu einer Beschädigung der Walze 40 führen könnte.

Nach dem Ausspritzvorgang wird mittels des Motors 51 die Rampe 42 wieder in der ersten Drehrichtung 52 gedreht, so dass, sobald die Walze 40 im Steigungsbereich 44 Kontakt mit der Rampenbahn 41 erlangt, ein weiteres Drehen dazu führt, dass der Mitnehmer 31 entlang der Längsrichtung der Kolbenstange 25 vom offenen Abgabeende 14 weg bewegt wird, wodurch die Druckfedern 32, 33 wieder gespannt werden und ihre maximale Spannung erreichen, wenn die Walze 40 das obere Plateau 45 erreicht. Diese Bewegung des Mitnehmers 31 führt aufgrund der mechanischen Verbindung des Mitnehmers 31 mit den Führungsstangen 29 und 30, der Platte 28 und der Kolbenstange 25 dazu, dass auch die Kolbenstange 25 und somit der Kolben 26 in einer Richtung weg vom offenen Abgabeende im Zylinder 13 bewegt und somit ein Unterdruck aufgebaut wird. Sobald der aufgebaute Unterdruck so groß ist, dass das Einlassventil 20 öffnet, wird das Fluid durch das Einlassventil 20 und die radialen Bohrungen 22 in den Zylinder 13 gesaugt, so dass der Zylinder 13 mit dem Fluid befüllt wird.

Wenn die Walze 40 (die auch als Nocken oder Rolle bezeichnet werden kann) das obere Plateau 45 erreicht hat, stoppt der Motor 51, so dass die Zylinder-Kolben-Anordnung 10 gespannt und damit die Applizierungsvorrichtung 1 bereit zum nächsten Applizierungsvorgang ist, der durch Betätigen des Auslösers 5 durchgeführt werden kann.

Die Platte 28, die Federn 32, 33 samt Führungsstangen 29, 30, der Mitnehmer 31 mit Walze 40, die Rampe 42 bilden zusammen mit Motor 51 und Kupplung 50 eine Spannvorrichtung S zum Spannen der Zylinder-Kolben-Anordnung 10.

Ferner umfasst die Applizierungsvorrichtung 1 eine Steuereinheit 54 zum Ansteuern des Motors 51 sowie allen weiteren elektrischen Komponente der Vorrichtung 1. In Fig. 3 ist eine Platine mit der Steuereinheit 54 gezeigt.

Wie bereits beschrieben wurde, wird zum Applizieren des Fluids ausgehend von der in Fig. 6 gezeigten Drehstellung der Rampe 42 die Rampe in der ersten Drehrichtung 52 weiter gedreht, so dass die Walze 40 vom oberen Plateau 45 über den Übergangsbereich 46 läuft und dann entlang der Sprungflanke 47 zum unteren Plateau 53 hin beschleunigt wird. Beim Überlaufen des Übergangsbereiches 46 tritt jedoch die Schwierigkeit auf (Fig. 12A - 12C), dass die Federkraft F der gespannten Federn 32, 33 neben einem tangentialen Anteil Ft einen dazu senkrechten Anteil Fs aufweist, der einen Anteil Fd umfasst, der in die gleiche Richtung weist wie die Kraft des Motors Fm zur Drehung der Rampe 42. Dadurch beschleunigt die über den Übergangsbereich 46 laufende Walze 40 die Drehung der Rampe 42 (zusätzlich zur durch den Motor 51 bedingten Drehung). Dies kann in nachteilhafter Weise dazu führen, dass der Motor 51 für diese zusätzliche Beschleunigung als Generator wirkt und einen Spannungspeak erzeugt, der die Ansteuerelektronik der Steuereinheit 54 schädigen kann. Darüber hinaus wirkt der Motor 51 dadurch als Bremse, so dass ein unerwünschter Bremseffekt bei der Drehung der Rampe 42 eintritt, der den Druckverlauf beim Applikationsvorgang in unerwünschter Weise ändert.

Daher ist die Kupplung 50 so ausgebildet, dass sie zum Drehen der Rampenbahn 41 in der ersten Drehrichtung 52 das vom Motor 51 bereitgestellte Drehmoment überträgt und gleichzeitig einen Freilauf entgegen der ersten Drehrichtung 52 aufweist, der so ausgelegt ist, dass er mindestens den Drehwinkelbereich (von α1 bis α2) abdeckt, der dem Übergangsbereich 46 entspricht (hier z.B. 7°).

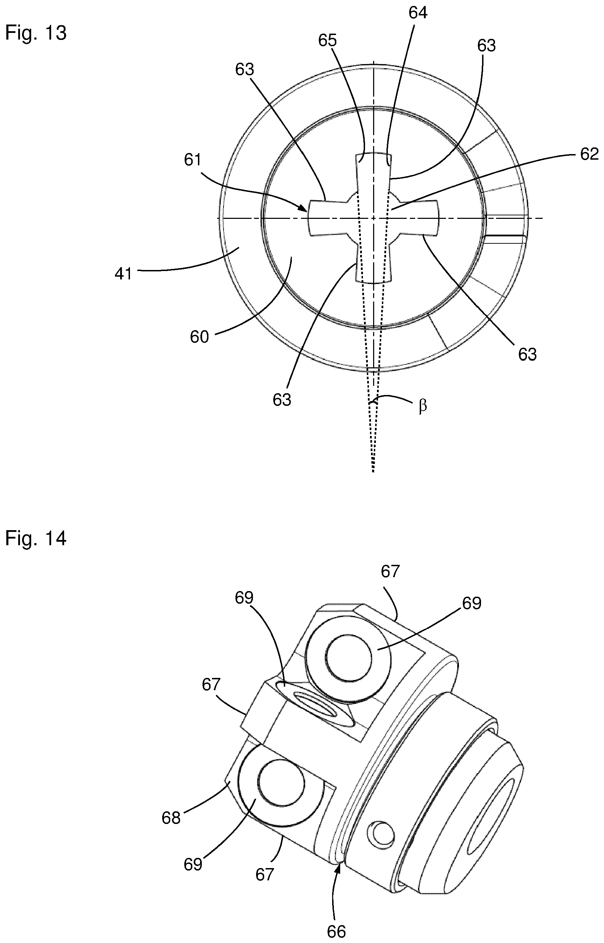

Zur Ausbildung der Kupplung 50 ist in einem Boden 60 der Rampe 42 eine sternförmige Ausnehmung 61 ausgebildet (Fig. 13). Die sternförmige Ausnehmung 61 umfasst einen mittleren Abschnitt 62 und vier sich davon erstreckende Arme 63, die in Umfangsrichtung jeweils 90° voneinander beabstandet sind. Wie in Fig. 13 schematisch für einen der Arme 63 eingezeichnet ist, sind die Seitenflächen 64, 65 der Arme 63 zueinander geneigt, so dass sie einen Winkel ß einschließen, der mindestens dem Drehwinkel des Übergangsbereiches 46 und somit hier 7° entspricht.

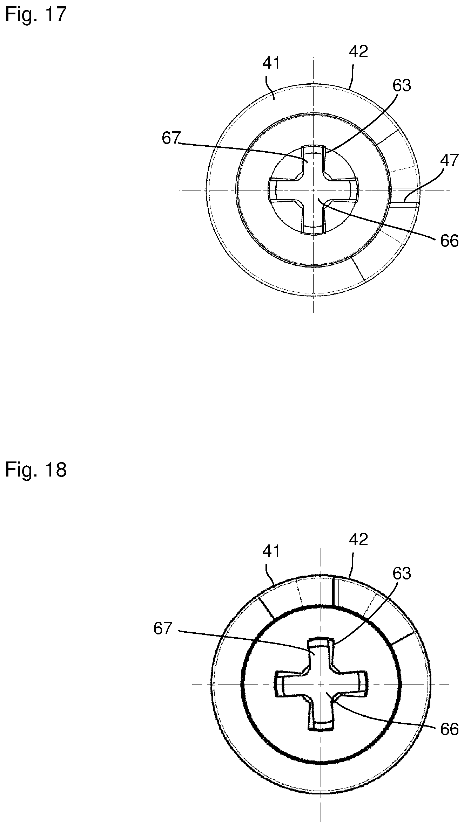

Ferner umfasst die Kupplung 50 ein mit dem Motor verbundenes Kupplungsteil 66, das vier sternförmig angeordnete Wandungen 67 aufweist, die jeweils um 90° in Umfangsrichtung voneinander beabstandet sind. An jeder Seitenfläche 68 jeder Wandung ist eine Feder 69 (hier Tellerfeder) angeordnet. Die Federn 69 dienen zum Unterstützen der Bewegung und zur Dämpfung. Die Wandungen 67 der sternförmigen Kontur des Kupplungsteils 66 sind in die sternförmige Ausnehmung 61 des Bodens 60 der Rampe 42 eingesetzt, wie in der Vorderansicht gemäß Fig. 17 gezeigt ist. Aufgrund der Federn 69 wird jede Wandung 67 im entsprechenden Arm 63 der sternförmigen Ausnehmung 61 zentriert, wenn über die Kupplung 50 kein Drehmoment übertragen wird.

Wenn mittels des Motors 51 die Walze 40 in der ersten Drehrichtung 52 gedreht wird, liegen die in der ersten Drehrichtung 52 gesehenen vorderen Seitenflächen 68 an der entsprechenden Seitenfläche 64 jedes Arms 63 an, wie in Fig. 18 gezeigt ist.

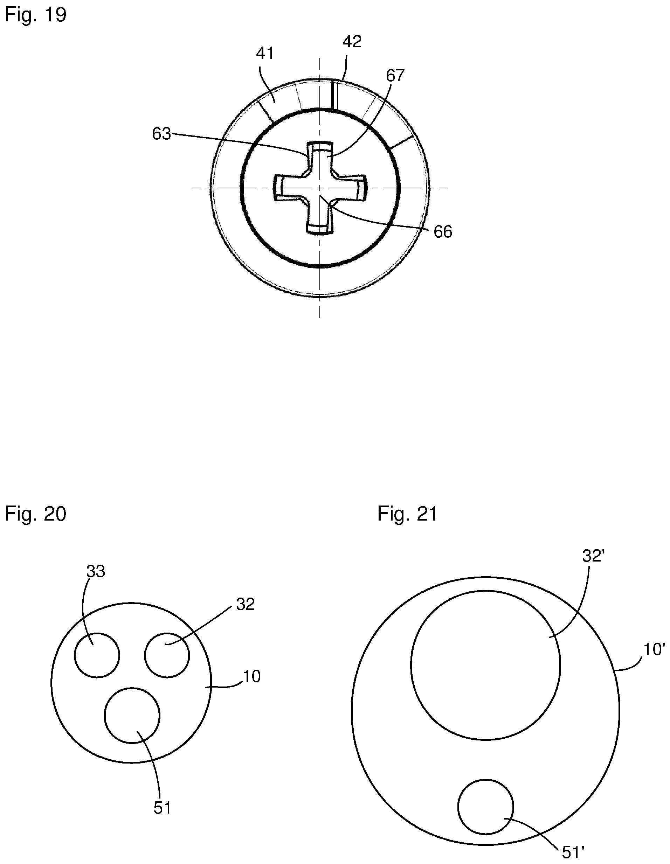

Wenn die Walze 40 ausgehend vom oberen Plateau 45 den Übergangsbereich 46 überläuft, wird aufgrund der beschriebenen Federkraft (hier die Komponente Fd) die Rampe 42 zusätzlich in der ersten Drehrichtung 52 beschleunigt, so dass aufgrund des vorgesehenen Freilaufes die Rampe 42 in der ersten Drehrichtung 52 schneller drehen kann als das mit dem Motor 51 verbundene Kupplungsteil 66. Dieser Freilauf endet, sobald die in erster Drehrichtung 52 gesehene hintere Seitenfläche 68 der jeweiligen Wandung 67 an der Seitenfläche 65 des entsprechenden Arms 63 der sternförmigen Ausnehmung 61 anliegt, wie in Fig. 19 gezeigt ist. Da der Freilauf so ausgelegt ist, dass er mindestens den gesamten Übergangsbereich 46 abdeckt, ist die Walze 40 über den gesamten Übergangsbereich 46 hinaus bewegt, sobald der Kontakt gemäß Fig. 19 vorliegt. Damit kann die Walze 40 sich frei entlang der Sprungflanke 47 bewegen und wird die unerwünschte Beschleunigung der Drehbewegung des Motors 51 beim Überlaufen des Übergangsbereiches 46 sicher vermieden.

In Fig. 20 ist eine schematische Vorderansicht gezeigt, die die räumliche Anordnung der Federn 32 und 33 sowie des Motors 51 zeigt. Die beiden Federn 32 und 33 sind über die Platte 28 parallel geschaltet, so dass sich ihre Federraten (Federkonstanten) addieren. Damit kann, wenn der Kolben 26 in seiner hinteren Endposition steht, die notwendige Kraft (Federkraft) bereitgestellt werden, die notwendig ist, um den Kolben 26 so stark zu beschleunigen, dass das abgebende Fluid bei einem Tier intramuskulär appliziert werden kann. Gleichzeitig kann der notwendige Bauraum für die Zylinder-Kolben-Anordnung 10 klein und kompakt gehalten werden. Wie ein Vergleich mit der Darstellung in Fig. 21 zeigt, bei der statt der beiden Federn 32 und 33 nur eine Feder 32' vorgesehen ist, würde dies zu einem größeren Bauraum für die entsprechende Zylinder-Kolben-Anordnung 10' führen, da diese einzelne Feder 33' einen größeren Durchmesser haben müsste, um die gleiche Federkraft bereitzustellen.

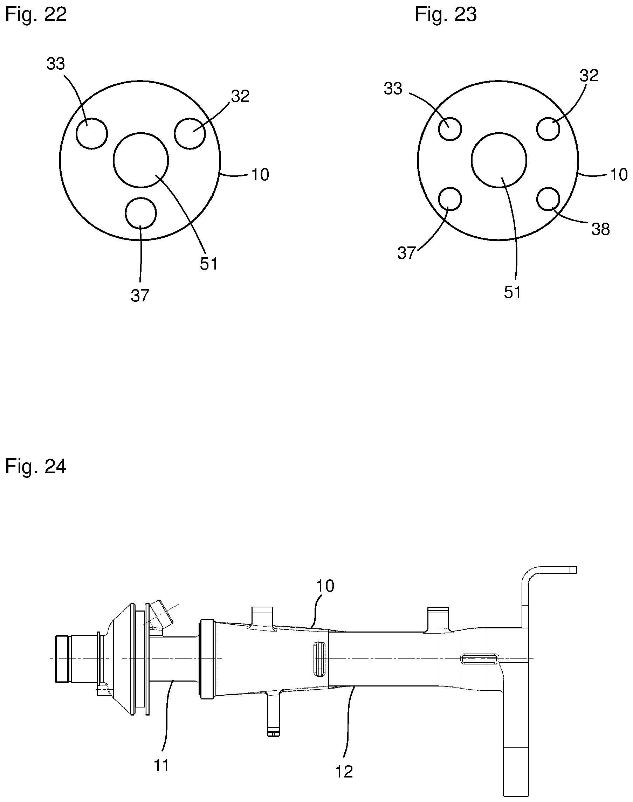

Natürlich ist es auch möglich, mehr als zwei Federn 32 und 33 parallel zu schalten. Wie in den schematischen Darstellungen von Fig. 22 und 23 ersichtlich ist, können z.B. drei oder vier Federn 32, 33, 37 und gegebenenfalls 38 vorgesehen werden, um eine kompakte Bauform zu realisieren. Die mehr als zwei (hier drei oder vier) Federn können bevorzugt symmetrisch zum Motor 51 angeordnet sein, wie in Fig. 22 und 23 gezeigt ist.

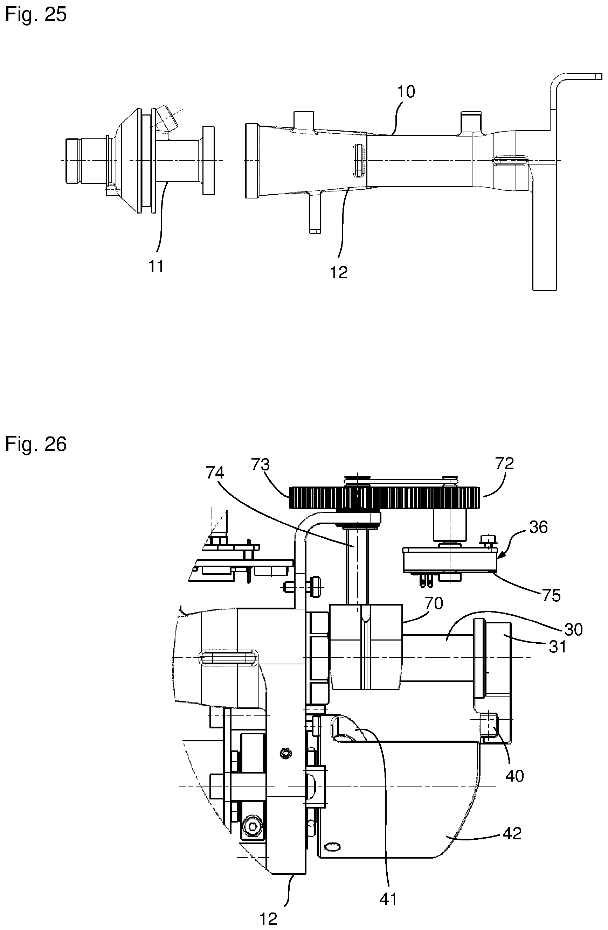

Wie bereits ausgeführt wurde, sind das Vorderteil 11 und das Hinterteil 12 zwei separate Teile, die miteinander verbunden sind, wie auch gut den Darstellungen in Fig. 24 und 25 zu entnehmen ist.

Bevorzugt sind das Vorderteil 11 und das Hinterteil 12 aus unterschiedlichen Werkstoffmaterialien gebildet. Da der vordere Abschnitt des Vorderteils 11 aus dem Gehäuse 2 herausschaut (Fig. 1), wird hierfür ein Material gewählt, das beispielsweise eine höhere Festigkeit aufweist als das Material für das Hinterteil und/oder das eine bessere Medienbeständigkeit als das Material des Hinterteils 12 aufweist.

So kann das Material des Vorderteils 11 Titan, Stahl oder Kunststoff (z.B. PEEK) aufweisen.

Für das Material des Hinterteils 12 wird insbesondere ein Material gewählt, das möglichst wenig Gewicht mit sich bringt. Bevorzugt sind hierbei Aluminium, Magnesium, Titan oder Kunststoff.

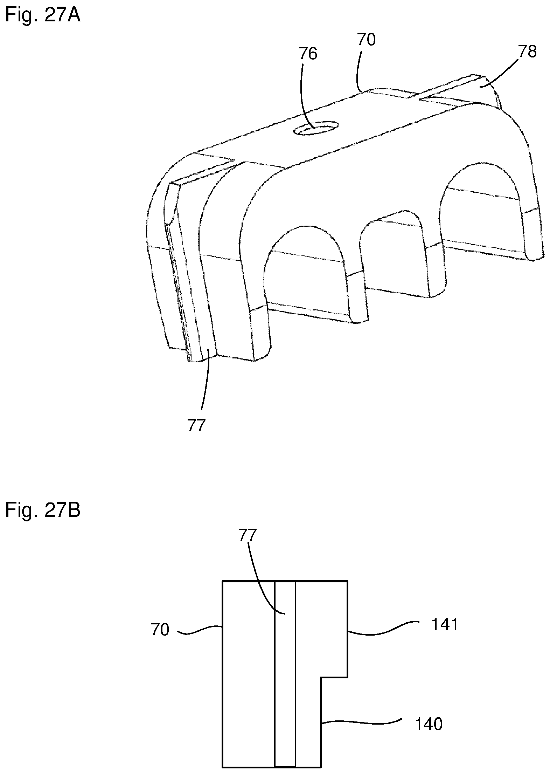

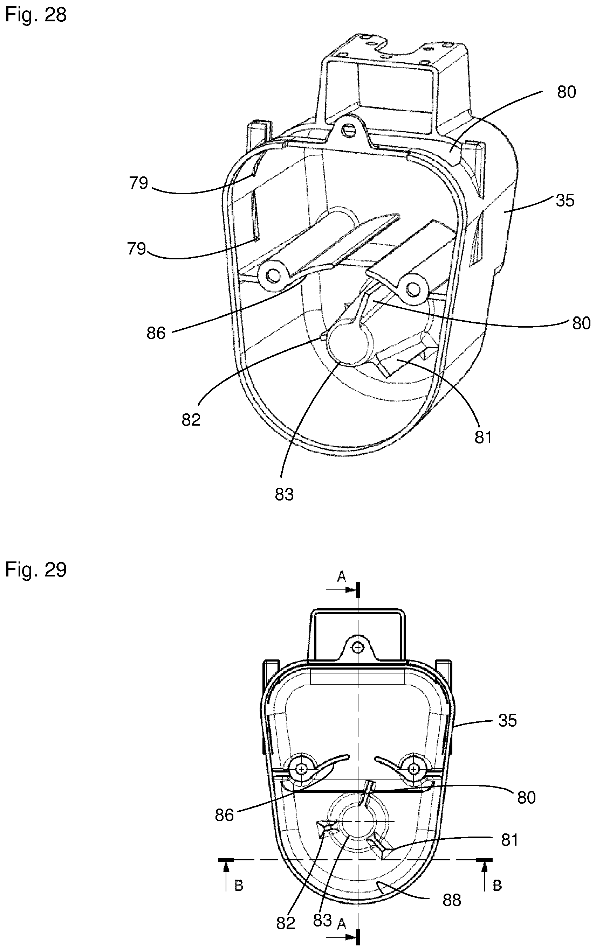

Die Dosiereinstelleinrichtung 36 umfasst, wie insbesondere der vergrößerten Detailschnittansicht in Fig. 26 sowie Fig. 27A zu entnehmen ist, einen Abstandshalter 70, in dem eine Gewindestange 71 eingeschraubt ist, die über ein erstes und zweites Zahnrad 72, 73 mit einer Welle 74 eines zweiten Motors 75 gekoppelt ist. Die Gewindestange 71 ist in eine Gewindebohrung 76 im Abstandshalter 70 (Fig. 3) eingeschraubt. Ferner umfasst der Abstandshalter 70 zwei seitlich vorstehende Führungsstege 77, 78 (Fig. 27A). Die Führungsstege 77, 78 sind in Führungsnuten 79 der Abdeckung 35 geführt. Die Führungsnuten 79 sind am besten in Fig. 28 erkennbar. Weiterhin umfasst die Abdeckung 35 eine Öffnung 80, durch die der Abstandshalter 70 bewegt werden kann.

In der Darstellung gemäß Fig. 3 steht der Abstandshalter 70 in seiner neutralen Position, in der er die Rückbewegung der Walze 40 und somit des Mitnehmers 31 vom oberen Plateau 45 über den Übergangsbereich 46 entlang der Sprungflanke 47 zum unteren Plateau 43 hin nicht beeinflusst. In Fig. 26 ist der Abstandshalter 70 hingegen in seine aktive Position bewegt worden, bei der er zwischen dem Mitnehmer 31 und dem hinteren Ende des Hinterteils 12 so positioniert ist, dass er einen Anschlag für den Mitnehmer 31 bildet. Die Bewegung des Abstandshalters von der in Fig. 3 gezeigten Position zu der in Fig. 26 gezeigten Position wird durch eine Drehung der Welle 74 erzeugt, wobei z.B. eine Rechtsdrehung der Welle 74 eine Bewegung von der in Fig. 3 gezeigten Position zu der in Fig. 6 gezeigten Position und eine Linksdrehung der Welle 74 eine entgegengesetzte Bewegung bewirkt. Natürlich kann die Dosiereinstelleinrichtung 36 auch so ausgelegt sein, dass die umgekehrten Drehrichtungen die gleichen Bewegungen bewirken. Wesentlich ist hier, dass mittels des zweiten Motors 75 die beiden Zahnräder 72 und 73 und damit die Welle 74 gedreht werden können, um diese Drehbewegung in eine Translationsbewegung des Abstandshalters 70 senkrecht zur Längsrichtung der Kolbenstange 25 umzusetzen. Damit kann der Abstandshalter 70 zwischen seiner aktiven Position und seiner neutralen Position hin und her bewegt werden.

Wenn nun der Abstandshalter 70 in der in Fig. 26 gezeigten aktiven Position ist, wird die Bewegung des Mitnehmers 31 in Längsrichtung der Kolbenstange 25, nachdem die Walze 40 den Übergangsbereich 46 überlaufen hat, verkürzt, da diese Bewegung nun endet, wenn der Mitnehmer 31 am Abstandshalter 70 anliegt. Die Ausdehnung des Abstandshalters 70 entlang der Längsrichtung der Kolbenstange 25 entspricht damit der Verkürzung des Kolbenhubs beim Applizieren des im Zylinder 13 befindlichen Fluids. Damit kann eine geringere Fluidmenge ausgespritzt werden, wodurch mit der Applizierungsvorrichtung 1 zwei unterschiedliche Dosierungen verabreicht werden können (hier z.B. 2 ml und 1 ml). Wenn die Dosierung verändert werden soll, muss lediglich, wenn die Walze 40 am oberen Plateau 45 ist, der Abstandshalter 70 in seine in Fig. 26 gezeigte aktive Position gebracht werden.

Der Abstandshalter 70 ist so ausgebildet, dass, wenn der Mitnehmer 31 an ihm anliegt, die Walze 40 keinen Kontakt zum Abstandshalter 70 hat. Damit wird verhindert, dass beim Abstoppen des Mitnehmers 31 durch den Abstandshalter 70 die Walze 40 beschädigt wird.

Mit dem beschriebenen Abstandshalter gemäß Fig. 27A ist es somit möglich, eine einzige geringere Dosierung einzustellen, wie beschrieben wurde. In Fig. 27B ist eine Abwandlung des Abstandshalters 70 gezeigt, mit der es möglich ist, zwei unterschiedliche geringere Dosierungen einzustellen, da der Abstandshalter 70 einen ersten Anschlagbereich 140 und einen zweiten Anschlagbereich 141 aufweist, die sich durch die Ausdehnung des Abstandshalters 70 entlang der ersten Richtung (in Fig. 27B von links nach rechts) unterschieden. Nachdem diese Ausdehnung der Verringerung des Kolbenhubs des Kolbens 26 beim Applizierungsvorgang entspricht, sind zwei unterschiedliche Verringerungen der Dosierung möglich. Wenn der Abstandshalter 70 so weit zwischen dem Mitnehmer 31 und dem hinteren Ende des Hinterteils 12 eingefahren ist, dass der Mitnehmer 31 durch den Abschnitt 140 beim Applizierungsvorgang gestoppt wird, liegt eine erste Reduzierung des Kolbenhubs vor. Wenn hingegen der Abstandshalter 70 so weit eingefahren ist, dass der Mitnehmer 31 beim Applizierungsvorgang am Bereich 141 anliegt, dann liegt eine zweite Verringerung des Kolbenhubs vor, die größer ist als die Verringerung durch den Abschnitt 140. Durch diese gestufte Ausbildung des Abstandshalters 70 ist es somit möglich, zwei unterschiedliche Verringerungen der Dosierung einzustellen.

Wie beispielsweise in Fig. 28 gut erkennbar ist, umfasst die Abdeckung 35 einen ersten, zweiten und dritten Abstreifer 80, 81, 82 die sich, im zusammengebauten Zustand gemäß Fig. 3, von einem hinteren Ende der Abdeckung 35 in Richtung zum Abgabeende der Applizierungsvorrichtung 1 erstrecken. Wie z.B. Fig. 28 zu entnehmen ist, sind die Abstreifer 80-82 an einem kegelstumpfförmigen Mitteilteil 83 ausgebildet und in Umfangsrichtung voneinander beabstandet. Das kegelstumpfförmige Mittelteil 83 verjüngt sich in Richtung zum Abgabeende hin, wie in Fig. 3 ersichtlich ist.

Im zusammengebauten Zustand erstreckt sich das kegelstumpfförmige Mittelteil 83 bis zum Boden 60 der Rampe 41. In gleicher Weise erstreckt sich der erste Abstreifer 80 bis zum Boden 60. In radialer Richtung erstreckt sich der erste Abstreifer 80 bis zur Innenseite 84 der Wandung 85, auf deren Stirnseite die Rampenbahn 42 ausgebildet ist (Fig. 6).

Der zweite Abstreifer 81 ist sowohl in axialer als in radialer Richtung kürzer als der erste Abstreifer 80. In gleicher Weise ist der dritte Abstreifer 82 in radialer und axialer Richtung kürzer als der zweite Abstreifer 81.

Ferner umfasst die Abdeckung 35 eine Zwischenwandung 86, in der ein sich in axialer Richtung erstreckender Schlitz ausgebildet ist, in der die Walze 40 zusammen mit ihrem Halterungsabschnitt des Mitnehmers 31 sich in axialer Richtung bewegen kann. Ansonsten umgibt die Zwischenwandung 86 zusammen mit dem unteren Abdeckungssteil 88 die Außenseite 89 der Wandung 85 im zusammengebauten Zustand. In diesem verbleibenden Raum zwischen der Abdeckung 35 und der Wandung 85 ist ein Schmiermittel (beispielsweise ein Fett) vorgesehen, das dazu dient, dass sich die Walze 40 möglichst leichtgängig dreht und mit möglichst wenig Reibung auf der Rampenbahn 41 geführt wird. Durch die Abstreifer 80 bis 82 wird das Fett, das nicht auf der Rampenbahn 41 bleibt, aufgrund der Relativbewegung zwischen Rampenbahn 41 und den Abstreifern 80-82 wieder Richtung Rampenbahn und Walze 40 bewegt, so dass eine dauerhafte Schmierung sichergestellt werden kann. Es wird somit das Schmiermittel, das sich unten in der Abdeckung 35 ansammelt, wieder zur Rampenbahn 41 und zur Walze 40 gefördert, so dass die gewünschte dauerhafte Schmierung sichergestellt ist.

In Fig. 34 ist eine vergrößerte Schnittdarstellung des Vorderteils 11 samt Kolben 26 und Teil der Kolbenstange 25 gezeigt. Der Zylinder 13 weist in seinem hinteren Bereich (abgewandt vom offenen Abgabeende 14) eine ringförmige Nut 90 auf, in der zur Abdichtung ein O-Ring 91 bzw. ein Dichtring 91 (z.B. eine Elastomerdichtung) eingesetzt ist. Ferner sind in der Nut 90 ein erster und ein zweiter Stützring (92, 93) so angeordnet, dass der Dichtring 91 zwischen beiden Stützringen 92 und 93 positioniert ist. Die Nut 90 und die Stützringe 92 und 93 sind so dimensioniert, dass der Spalt zwischen den Stützringen 92 und 93 und der Kolbenstange 25 kleiner ist als zwischen der Innenseite des Zylinders 13 und der Kolbenstange 25. Durch die Stützringe, die z.B. aus PTFE oder andere Kunststoffe hergestellt sein können, wird sicher vermieden, dass ein Teil des Dichtringes 91 aufgrund des bei Bewegung der Kolbenstange 95 aufgebauten Drucks bzw. Unterdrucks in den Spalt zwischen der Kolbenstange 25 und der Innenseite des Zylinders 13 extrudiert wird, was den Dichtring 91 zerstören würde.

Der zweite Stützring 93 verhindert die beschriebene Spaltextrusion bei einer Bewegung der Kolbenstange 25 zum offenen Abgabeende 14 hin und somit beim Applizieren des Fluids. Der erste Stützring 92 verhindert die unerwünschte Spaltextrusion bei der entgegengesetzten Bewegung und somit beim Füllen des Zylinders 13 mit dem Fluid.

Wie in Fig. 34 erkennbar ist, weist die Düse 16 eine sich verjüngende Durchgangsbohrung 95 auf, durch die das Fluid beim Applizieren abgegeben wird. Die notwendige Durchgangsbohrung 95 kann auch in einem Einsatz 96 ausgebildet sein, wie in Fig. 35 gezeigt ist, der dann in den restlichen Düsengrundkörper 97 einzuschrauben ist. Der Einsatz 96 umfasst einen Basiskörper 98 mit einem Außengewinde, der am distalen Ende einen Aufnahmebereich 99 umfasst. In den Aufnahmebereich 99 ist ein Saphirelement 100 eingesetzt, in dem der letzte Abschnitt der Durchgangsbohrung 95 ausgebildet ist. Wie der Darstellung in Fig. 35 entnommen werden kann, ist der Durchmesser des letzten Abschnitts der Durchgangsbohrung 95 am geringsten bzw. geringer als der Durchmesser der in dem Basiskörper 98 ausgebildeten Abschnitte der Durchgangsbohrung 95. Damit wird vorteilhaft erreicht, dass der notwendige sehr geringe Durchmesser der Durchgangsbohrung 95 an ihrem distalen Ende zuverlässig herstellbar ist, da der Abschnitt der Durchgangsbohrung 95 im Saphirelement 100 genauer gefertigt werden kann als eine Bohrung im Basiskörper 98, der aus Metall hergestellt ist. Der Durchmesser des letzten Abschnitts der Durchgangsbohrung 95 im Saphirelement 100 kann z.B. im Bereich von 0,30 bis 0,38 mm liegen, wobei die Fertigungstoleranz nicht größer als 0,02 mm sein soll.

Da die Einheit aus Kolbenstange 26, Platte 28 und Führungsstangen 29, 30 relativ lang ist und beim Applizieren des Fluids hohe Kräfte wirken, muss sichergestellt werden, dass sich die Kolbenstange 26 im Zylinder 13 frei bewegen kann und z.B. nicht verkantet. Dazu sollte die Kolbenstange 26 beispielsweise möglichst parallel zu den Führungsstangen 29, 30 ausgerichtet sein und dies auch über lange Zeit bei der Benutzung der Applizierungsvorrichtung 1 bestehen bleiben.

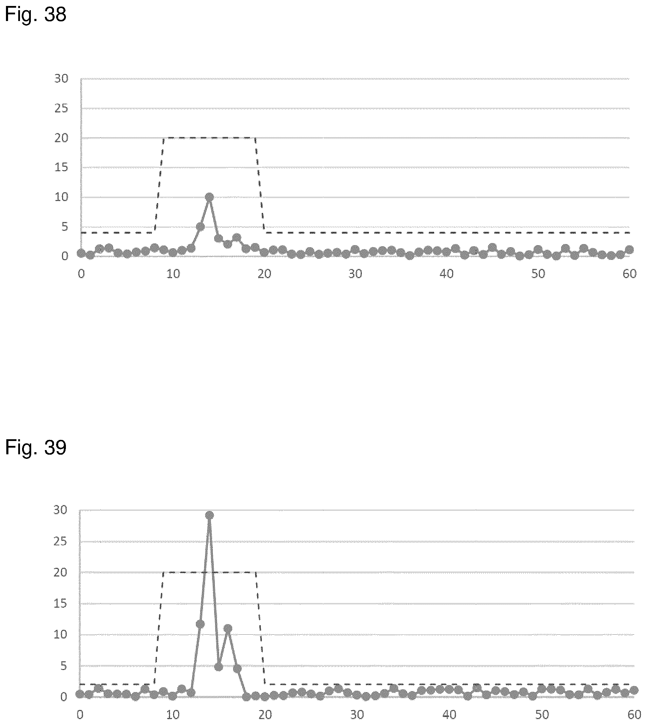

Daher ist die Kolbenstange 25 nicht absolut starr mit der Platte 28 verbunden. Die Verbindung ist so ausgelegt, dass ein Verkippen oder Drehen der Kolbenstange 26 gegenüber der Platte 28 möglich ist. Die Kolbenstange 25 ist somit über ein Drehgelenk mit der Platte 28 verbunden. Wie den Darstellungen in Fig. 36 und 39 zu entnehmen ist, ist zwischen dem hinteren Ende 27 der Kolbenstange 25 und der Platte 28 eine erste Beilagscheibe 110 vorgesehen. Ferner ist in das hintere Ende 27 eine Fixierschraube 111, die durch eine entsprechende Bohrung in der Platte 28 läuft, eingeschraubt. Zwischen einem Kopf 112 der Fixierschraube 111 sind eine zweite und eine dritte Beilagscheibe 113, 114 angeordnet. Um die gewünschte Drehbarkeit bereitzustellen, ist das hintere Ende 27 abgerundet (hier z.B. kugelförmig) und ist die zu dem hinteren Ende 27 weisende Seite der ersten Beilagscheibe 110 entsprechend konkav ausgebildet, so dass diese Seite ein Bett für das hintere Ende 27 bildet. Wie der Darstellung in Fig. 37 zu entnehmen ist, sitzt die erste Beilagscheibe 110 in einer Vertiefung in der Platte 28, so dass sich die erste Beilagscheibe 110 quer zur Längsrichtung der Kolbenstange 25 nicht bewegen kann. Die dem hinteren Ende 27 abgewandte Seite der ersten Beilagscheibe 110 ist plan ausgebildet, da der entsprechende Boden der Ausnehmung in der Platte 28 auch plan ist. Somit kann die erste Beilagscheibe auch als konkav-plan bezeichnet werden.

Die zweite und dritte Beilagscheibe 113 und 114 sind so ausgebildet, dass die einander zugewandten Seiten wiederum gekrümmt sind. Dabei weist die der dritten Beilagscheibe 114 zugewandte Seite der zweiten Beilagscheibe 113 eine konvexe Krümmung auf. Die der zweiten Beilagscheibe 113 zugewandte Seite der dritten Beilagscheibe 114 ist entsprechend konkav gekrümmt. Die anderen Seiten der zweiten und dritten Beilagscheibe 113, 114 sind plan ausgebildet. Der Kopf 112 der Fixierschraube 111 drückt die dritte Beilagscheibe 114 auf die zweite Beilagscheibe 113, die dadurch gegen die von dem hinteren Ende 27 weg weisenden Seite der Platte 28 gedrückt wird. Die zweite Beilagscheibe 113 ist somit plan-konvex und die dritte Beilagscheibe 114 ist somit konkav-plan ausgebildet.

Durch die gewählten Abmessungen und Krümmungen liegt der Drehpunkt 115 für die Drehung der Kolbenstange 25 relativ zur Platte 28 beabstandet zur Platte 28 und auf der Seite des Schraubenkopfs 112.

Da die beschriebene Verbindung ein Drehen der Kolbenstange 25 relativ zur Platte 28 ermöglicht, kann sichergestellt werden, dass die Kolbenstange 25 stets im Zylinder 13 ohne Verkeilen bewegt werden kann.

Der Motor 51 kann als Elektromotor und insbesondere als bürstenloser Elektromotor ausgebildet sein. Damit wird die Haltbarkeit der Applizierungsvorrichtung 1 verbessert, da bei Elektromotor mit Bürsten die Schwierigkeit auftreten kann, dass aufgrund der auftretenden Erschütterungen beim Applizieren des Fluids die Bürsten brechen können.

Um zu erkennen, ob Fluid im Zylinder 13 bei der in Fig. 3 gezeigten Position des Kolbens 26 ist, ist ein Sensor 55 vorgesehen, der bei dem hier beschriebenen Ausführungsbeispiel vor dem weiteren Rückschlagventil 20 (das auch als Einlassventil bezeichnet werden kann) vorgesehen ist. Der Sensor 55 kann beispielsweise Luft von Flüssigkeit unterscheiden, so dass dadurch vermieden werden kann, dass die Applizierungsvorrichtung 1 einen Applikationsvorgang durchführt, wenn keine Flüssigkeit im Zylinder 13 vorhanden ist. Damit kann eine Beschädigung der Applizierungsvorrichtung 1 verhindert werden, da diese so ausgelegt ist, dass die Flüssigkeit die Bewegung der Kolbenstange 25 bzw. des Kolbens 26 beim Applizieren hin zum offenen Abgabeende dämpft. Wenn keine Flüssigkeit im Zylinder 13 ist, entfällt diese dämpfende Funktion, wodurch mechanische Beschädigungen z.B. der Kolbenstange 25, der Verbindung der Kolbenstange 25 mit der Platte 28 oder an den Führungsstangen 29, 30 auftreten können. Der Sensor 55 kann als Spannungssensor, als kapazitiver Sensor oder beispielsweise als Lichtschranke ausgebildet sein.

Das Gehäuse 2 kann einen leuchtenden Bereich 120 aufweisen (Fig. 1), der in unterschiedlichen Farben leuchten kann. Der Bereich 120 kann z.B. streifenförmig sein oder auch jede andere Form aufweisen. Mit den unterschiedlichen Farben können Benutzerinformationen über den Zustand der Applizierungsvorrichtung 1 mitgeteilt werden. So kann z.B. mit einer ersten Farbe (beispielsweise die Farbe Rot) dem Benutzer mitgeteilt werden, dass die Vorrichtung 1 nicht bereit ist, eingesetzt zu werden. Mit einer zweiten Farbe kann mitgeteilt werden, dass sie grundsätzlich zum Betrieb bereit ist. Mit einer dritten Farbe kann mitgeteilt werden, dass die Zylinder-Kolben-Anordnung 10 gespannt ist und durch Betätigen des Auslösers 5 ein Applizierungsvorgang durchgeführt werden kann. Mit einer vierten Farbe (beispielsweise grün) kann dem Benutzer mitgeteilt werden, dass der Applizierungsvorgang erfolgreich war. Ferner kann über eine weitere Farbe dem Benutzer mitgeteilt werden, dass ein Fehlerzustand vorliegt. Natürlich können die beschriebenen Informationen nicht nur über unterschiedliche Farben, sondern auch über gleiche Farben mitgeteilt werden, wenn die Unterschiede z.B. durch unterschiedliches Blinken dargestellt werden. Es ist ferner möglich, nicht dieses beschriebene optische Feedback dem Benutzer bereitzustellen, sondern ein haptisches oder akustisches Feedback bereitzustellen. Natürlich können das optische, haptische und akustische Feedback auch kombiniert werden.

Ferner kann die Applizierungsvorrichtung 1 einen Beschleunigungssensor 130 aufweisen, der beispielhaft nur in Fig. 3 eingezeichnet ist. Da sich die gemessenen Beschleunigungswerte für einen korrekten Applizierungsvorgang von denen eines nicht korrekten Applizierungsvorgangs unterscheiden, kann anhand der gemessenen Werte entschieden werden, ob ein Applizierungsvorgang erfolgreich war oder nicht. In Fig. 38 sind die gemessenen Beschleunigungswerte für einen erfolgreichen Applizierungsvorgang entlang der y-Achse in g (= Fallbeschleunigung) über die Zeit entlang der x-Achse (in ms) aufgetragen. Die gemessenen Beschleunigungswerte sind als Punkte dargestellt, die durch eine Linie verbunden sind. Eine Prüfkurve ist gestrichelt eingezeichnet. Wenn die Beschleunigungswerte unterhalb der Werte der Prüfkurve liegen, wird entschieden, dass der Applizierungsvorgang erfolgreich war.

In Fig. 39 ist ein Beispiel eines Applizierungsvorgangs (der nachfolgend auch als Schuss bezeichnet wird) gezeigt, der nicht erfolgreich war. Die Beschleunigungswerte übersteigen den Maximalwert der Prüfkurve, so dass von einem nicht erfolgreichen Schuss auszugehen ist.

Ferner kann zur Beurteilung der Qualität des Schusses die Stromaufnahme des Motors 51 gemessen und ausgewertet werden.

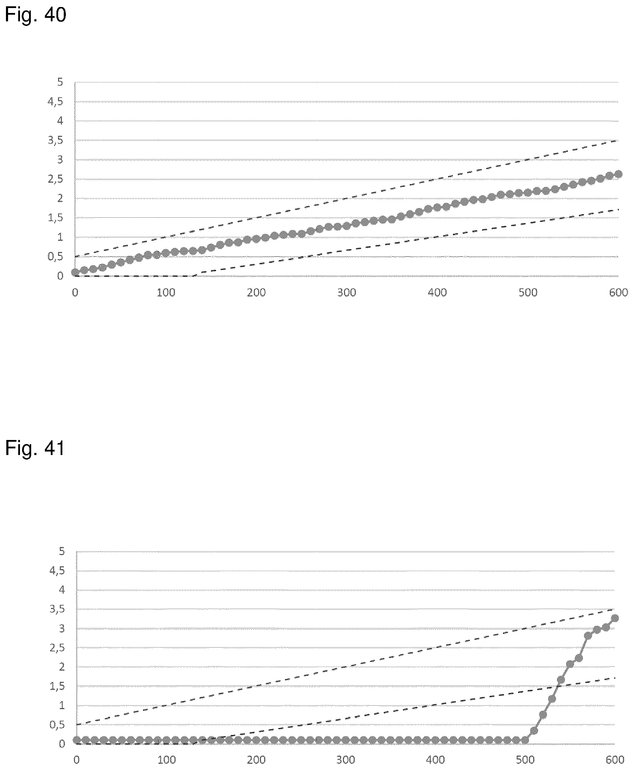

In Fig. 40 ist die gemessene Stromaufnahme für den Lade- und Schussvorgang der Applizierungsvorrichtung 1 gezeigt, wobei die gemessenen Stromwerte in A (= Ampere) als Punkte eingezeichnet sind, die durch eine Linie verbunden sind. Der Stromwert ist entlang der y-Achse (über die Zeit in ms entlang der x-Achse) auftragen. Ein erfolgreicher Lade- und Schussvorgang liegt dann vor, wenn die gemessenen Stromwerte kleiner als die obere Grenzwertkurve und größer als die untere Grenzwertkurve (beide Kurven sind gestrichelt eingezeichnet) liegen.

Bei einem nicht erfolgreichen Schuss liegt die gemessene Stromaufnahme außerhalb des durch die beiden Grenzwertkurven begrenzten Bereiches, wie in Fig. 41 dargestellt ist. In diesem Fall war der Applizierungsvorgang nicht erfolgreich.

Anstatt oder zusätzlich zum Beschleunigungssensor 130 kann ein Sensor 131 zum Messen von Schall bzw. Tönen (z.B. ein Mikrofon) vorgesehen sein, der schematisch in Fig. 3 dargestellt ist. Anhand der Tonhöhen während des Abgabevorgangs kann z.B. ermittelt werden, ob der Applizierungsvorgang erfolgreich war oder nicht. Wenn der zeitliche Verlauf der gemessenen Tonhöhe z.B. höher ist als eine vorgegebene obere Grenze bzw. ein zeitlicher Verlauf der oberen Grenze ist, wird der Applizierungsvorgang als nicht erfolgreich bewertet. Auch kann der zeitliche Verlauf der gemessenen Tonhöhe z.B. einen zeitlichen Verlauf einer unteren Grenze unterschreiten, was auf einen fehlerhaften Applizierungsvorgang schließen lässt. Man kann beispielsweise auch ein gemessenes Frequenzspektrum als Kenngröße auswerten, das einen zeitlichen Sollverlauf erfüllen muss, um den Applizierungsvorgang als erfolgreich zu bewerten. In gleicher Weise kann der zeitliche Verlauf der Intensität (bzw. Lautstärke) als Kenngröße genutzt werden, die wiederrum einen zeitlichen Sollverlauf erfüllen muss.

Natürlich können auch mehrere der beschriebenen Kenngrößen genutzt werden, um die Bewertung des Applizierungsvorgangs durchzuführen. Dabei kann z.B. nur der Abgabevorgang, nur der Spannvorgang oder Spann- und Abgabevorgang zusammen gemessen und bewertet werden.

Die Steuereinheit 54 kann die beschriebene Messung und Auswertung der Kenngrößen durchführen, um zu entscheiden, ob der Applizierungsvorgang erfolgreich war oder nicht. Abhängig von der Entscheidung, kann z.B. die Steuereinheit ein optisches, haptisches und/oder akustisches Feedback in der beschriebenen Art und Weise erzeugen.

## Patentansprüche

1. Vorrichtung zum Applizieren eines Fluids, mit
einem Zylinder (13), der ein offenes Abgabeende (14) aufweist,
einem im Zylinder (13) zwischen einer vorderen und hinteren Endposition verschiebbaren Kolben (26), der mit einer Kolbenstange (25) verbunden ist, die entlang einer ersten Richtung über ein dem offenen Abgabeende (14) entgegengesetztes hinteres Ende des Zylinders (13) heraussteht,
einem das offene Abgabeende verschließenden Rückschlagventil (15) und
einer mit der Kolbenstange (25) verbundenen Spannvorrichtung (S);
wobei die Spannvorrichtung (S) die Kolbenstange (25) in einem Spannvorgang entlang der ersten Richtung bewegen kann, bis der Kolben (26) in seiner hinteren Endposition steht, um den Zylinder (13) dadurch mit dem zu applizierenden Fluid zu befüllen und die Kolbenstange (25) zum offenen Abgabeende (14) hin vorzuspannen, und
wobei die Spannvorrichtung (S), wenn der Kolben (26) in seiner hinteren Endposition steht, die Kolbenstange (25) in einem Abgabevorgang freigeben kann, so dass der Kolben (26) aufgrund der anliegenden Vorspannung entgegen der ersten Richtung zum offenen Abgabeende (14) hin bewegt und dabei Fluid im Zylinder (13) über das Rückschlagventil (15) zum Applizieren abgegeben wird,
**dadurch gekennzeichnet, dass**
die Spannvorrichtung (S) eine mittels eines Motors (51) drehbare Rampe (42) mit einer sich entlang einer Schraubenlinie erstreckenden Rampenbahn (41) aufweist, wobei die Rampenbahn (41) von einem ersten Plateau (43) entlang eines Steigungsbereiches (44) zu einem zweiten Plateau (45) ansteigt und vom zweiten Plateau (45) über eine Sprungflanke (47) zum ersten Plateau (43) abfällt, wobei die Rampenbahn einen das zweite Plateau (45) und die Sprungflanke (47) verbindenden Übergangsbereich (46) aufweist,
wobei die Spannvorrichtung (S) ferner eine die Rampenbahn (41) kontaktierende Walze (40), die in einem Mitnehmer (31), der mit der Kolbenstange (25) verbunden ist, drehbar gelagert ist, aufweist, so dass bei Drehung der Rampe (42) entlang einer ersten Drehrichtung (52) die Rampenbahn (41) unter der sich dadurch drehenden Walze (40) durchläuft,
wobei für den Spannvorgang vorgesehen ist, dass
die Rampenbahn (41) entlang der ersten Drehrichtung (52) so gedreht wird, dass die Walze (40) auf dem Steigungsbereich (44) bis zum zweiten Plateau (45) läuft und dadurch der Kolben (26) in seine hintere Endposition bewegt wird, wobei für den Abgabevorgang vorgesehen ist, dass
die Rampenbahn (41) ausgehend von einem Kontakt der Walze (40) mit dem zweiten Plateau (45) so entlang der ersten Drehrichtung (52) gedreht wird, bis die Walze (40) über den Übergangsbereich (46) läuft und aufgrund der Vorspannung zum ersten Plateau (43) hin beschleunigt und dadurch der Kolben (26) zum offenen Abgabeende (14) hin bewegt wird,
wobei die Kolbenstange (25) über ein Gelenk mit dem Mitnehmer (31) verbunden ist.

2. Vorrichtung nach Anspruch 1, wobei
zur Ausbildung des Gelenks das dem Kolben (26) abgewandte Ende der Kolbenstange (25) abgerundet ist und bewegbar in einem Bett (110) gelagert ist.

3. Vorrichtung nach einem der vorhergehenden Ansprüche, wobei
das Bett (110) an einem Verbindungsteil (28) ausgebildet ist, das mittels einer in das abgerundete Ende eingeschraubten Schraube (111) gegen das abgerundete Ende der Kolbenstange (25) drückt.

4. Vorrichtung nach einem der obigen Ansprüche, wobei
das Gelenk zwei aufeinander angeordnete Beilagscheiben aufweist, deren einander zugewandte Seiten gekrümmt ausgebildet sind, so dass sie bei Drehung der Kolbenstange (25) gegeneinander bewegt werden.

5. Vorrichtung nach einem der obigen Ansprüche, wobei
das Gelenk als Drehgelenk ausgebildet ist.

6. Vorrichtung nach einem der obigen Ansprüche, wobei
das Gelenk als Gelenk mit genau einem Freiheitsgrad ausgebildet ist.

7. Vorrichtung nach einem der Ansprüche 1 bis 4, wobei
das Gelenk eine translatorische Bewegung quer zur Längsrichtung der Kolbenstange (25) zulässt.

## Claims

1. A device for administering a fluid, comprising
a cylinder (13), which has an open dispensing end (14),
a piston (26), which is displaceable in the cylinder (13) between a front end position and a rear end position and is connected to a piston rod (25) which, along a first direction, protrudes from a rear end of the cylinder (13) opposite the open dispensing end (14), a nonreturn valve (15) closing the open dispensing end, and
a tensioning device (S) connected to the piston rod (25);
wherein the tensioning device (S) can move the piston rod (25) along the first direction in a tensioning procedure until the piston (26) is in its rear end position, in order thereby to fill the cylinder (13) with the fluid to be administered and to pretension the piston rod (25) toward the open dispensing end (14), and
wherein the tensioning device (S), when the piston (26) is in its rear end position, can release the piston rod (25) in a dispensing procedure such that, owing to the pretension which is present, the piston (26) is moved counter to the first direction toward the open dispensing end (14) and, in the process, fluid in the cylinder (13) is dispensed via the nonreturn valve (15) for administration,
**characterized in that**
the tensioning device (S) has a ramp (42) which is rotatable by means of a motor (51) and has a ramp track (41) extending along a helical line,
wherein the ramp track (41) ascends from a first plateau (43) along a region of inclination (44) to a second plateau (45) and descends from the second plateau (45) to the first plateau (43) via a transition flank (47), wherein the ramp track has a transfer region (46) connecting the second plateau (45) and the transition flank (47),
wherein the tensioning device (S) moreover has a roller (40) which is in contact with the ramp track (41) and which is mounted rotatably in a driver (31), the latter being connected to the piston rod (25), and therefore, upon rotation of the ramp (42) along a first rotation direction (52), the ramp track (41) runs below the thus rotating roller (40),
wherein, for the tensioning procedure, the ramp track (41) is rotated along the first rotation direction (52) such that the roller (40) runs on the region of inclination (44) as far as the second plateau (45) and the piston (26) is thereby moved to its rear end position, wherein, for the dispensing procedure, starting from a contact of the roller (40) with the second plateau (45), the ramp track (41) is rotated along the first rotation direction (52) until the roller (40) runs over the transfer region (46) and, on account of the tensioning, is accelerated toward the first plateau (43) and, as a result, the piston (26) is moved toward the open dispensing end (14),
wherein the piston rod (25) is connected to the driver (31) via a joint.

2. The device as claimed in claim 1, wherein
in order to form the joint, that end of the piston rod (25) which faces away from the piston (26) is rounded and is mounted movably in a bed (110).

3. The device as claimed in either of the preceding claims, wherein
the bed (110) is formed on a connecting part (28) which, by means of a screw (111) screwed into the rounded end, presses against the rounded end of the piston rod (25).

4. The device as claimed in one of the preceding claims, wherein
the joint has two washers arranged one on the other and the mutually facing sides of which are curved such that they move against each other during rotation of the piston rod (25).

5. The device as claimed in one of the preceding claims, wherein
the joint is designed as a rotary joint.

6. The device as claimed in one of the preceding claims, wherein
the joint is designed as a joint with precisely one degree of freedom.

7. The device as claimed in one of claims 1 to 4, wherein
the joint permits a translational movement transversely with respect to the longitudinal direction of the piston rod (25).

## Revendications

1. Dispositif pour appliquer un fluide, comprenant
un cylindre (13) qui possède une extrémité de sortie ouverte (14),
un piston (26) pouvant coulisser dans le cylindre (13) entre une position avant et une position arrière et relié à une tige de piston (25) qui dépasse dans une première direction au-delà d'une extrémité arrière du cylindre (13) opposée à l'extrémité de sortie ouverte (14),
un clapet anti-retour (15) fermant l'extrémité de sortie ouverte et
un dispositif de serrage (S) relié à la tige de piston (25);
le dispositif de serrage (S) pouvant déplacer la tige de piston (25) dans une opération de serrage le long de la première direction jusqu'à ce que le piston (26) se trouve dans sa position arrière finale, afin de remplir ainsi le cylindre (13) avec le fluide à appliquer et de précontraindre la tige de piston (25) vers l'extrémité de sortie ouverte (14), et
le dispositif de serrage (S), lorsque le piston (26) se trouve dans sa position arrière extrême, peut libérer la tige de piston (25) lors d'une opération de distribution, de sorte que le piston (26), en raison de la précontrainte appliquée, se déplace dans le sens opposé à la première direction vers l'extrémité de distribution ouverte (14) et que le fluide contenu dans le cylindre (13) est alors distribué via le clapet anti-retour (15) pour être appliqué,
**caractérisé en ce que**
le dispositif de serrage (S) possède une rampe (42) pouvant être tournée au moyen d'un moteur (51) avec une trajectoire de rampe (41) s'étendant le long d'une ligne hélicoïdale,
la piste en rampe (41) montant depuis un premier plateau (43) le long d'une zone de pente (44) jusqu'à un deuxième plateau (45) et descendant depuis le deuxième plateau (45) vers le premier plateau (43) via un flanc de saut (47), la rampe (41) comportant une zone de transition (46) reliant le deuxième plateau (45) et le flanc de saut (47),
le dispositif de serrage (S) comportant en outre un rouleau (40) en contact avec la rampe (41), qui est monté de manière rotative dans un entraîneur (31) relié à la tige de piston (25) de telle sorte que, lors de la rotation de la rampe (42) dans un premier sens de rotation (52), la rampe (41) passe sous le rouleau (40) qui tourne alors
l'opération de serrage étant prévue de telle sorte que la rampe (41) tourne dans le premier sens de rotation (52) de manière que le rouleau (40) se déplace sur la zone inclinée (44) jusqu'au deuxième plateau (45) et que le piston (26) soit ainsi amené dans sa position arrière finale,
l'opération de distribution étant prévue de telle sorte que la rampe (41), à partir d'un contact du rouleau (40) avec le deuxième plateau (45), soit tournée le long du premier sens de rotation (52) jusqu'à ce que le rouleau (40) passe sur la zone de transition (46) et, en raison de la précontrainte, accélère vers le premier plateau (43) et déplace ainsi le piston (26) vers l'extrémité de distribution ouverte (14),
la tige de piston (25) étant reliée à l'entraîneur (31) par une articulation.

2. Dispositif selon la revendication 1, dans lequel
pour former l'articulation, l'extrémité de la tige de piston (25) opposée au piston (26) est arrondie et montée de manière mobile dans un lit (110).

3. Dispositif selon l'une des revendications précédentes, dans lequel
le lit (110) est formé sur une pièce de liaison (28) qui, au moyen d'une vis (111) vissée dans l'extrémité arrondie, appuie contre l'extrémité arrondie de la tige de piston (25).

4. Dispositif selon l'une des revendications précédentes, dans lequel
l'articulation possède deux rondelles superposées dont les côtés tournés l'un vers l'autre sont de forme curviligne, de sorte qu'elles sont déplacées l'une par rapport à l'autre lors de la rotation de la tige de piston (25).

5. Dispositif selon l'une des revendications précédentes, dans lequel
l'articulation est réalisée sous forme d'articulation pivotante.

6. Dispositif selon l'une des revendications précédentes, dans lequel
l'articulation est conçue comme une articulation avec exactement un degré de liberté.

7. Dispositif selon l'une des revendications 1 à 4, dans lequel
l'articulation permet un mouvement de translation transversalement à la direction longitudinale de la tige de piston (25).
